(19) Europäisches Patentamt
European Patent Office
Office européen des brevets

(11) **EP 3 900 546 A1**

(12) **EUROPEAN PATENT APPLICATION**
published in accordance with Art. 153(4) EPC

(43) Date of publication:
**27.10.2021 Bulletin 2021/43**

(51) Int Cl.:
*A23K 10/30* [(2016.01)]    *A23K 50/75* [(2016.01)]

(21) Application number: **19889039.2**

(22) Date of filing: **20.12.2019**

(86) International application number:
**PCT/BR2019/050557**

(87) International publication number:
**WO 2020/124191 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.12.2018 BR 102018076813**

(71) Applicant: **Suzano S.A.**
**41810-012 Salvador - BA (BR)**

(72) Inventors:
• **FERNANDES NUNES DA SILVA, Vinícius**
**13465-070 Americana, SP (BR)**
• **BUENO DA SILVA, Henrique**
**Pouso-Alegre, MG (BR)**

(74) Representative: **Cabinet Becker et Associés**
**25, rue Louis le Grand**
**75002 Paris (FR)**

(54) **FOOD AND ADDITIVE COMPOSITIONS, AND ALSO USES OF LIGNIN AND THE ADDITIVE COMPOSITION**

(57)    The present invention relates to a food composition comprising lignin as a food additive, to a food composition comprising an additive composition which in turn comprises lignin, to an additive composition comprising lignin, as well as to the uses of lignin and the additive composition.

Also disclosed are benefits of the food composition comprising lignin or comprising an additive composition comprising lignin for animals as well as for products derived thereof.

**Figure 01**

p-coumaryl alcohol    coniferyl alcohol    synaphyl alcohol

**Description**

FIELD OF THE INVENTION

The present invention relates to a food composition comprising lignin as a food additive, to a food composition comprising an additive composition which in turn comprises lignin, to an additive composition comprising lignin, as well as to the uses of lignin and the additive composition.

Also disclosed are benefits of the food composition comprising lignin or comprising an additive composition comprising lignin for animals as well as for products derived thereof.

BACKGROUND OF THE INVENTION

There is a constant search in the food industry for food additives to be used in animal diet for nutritional supplementation and for improving animal performance. Antibiotics are additives commonly used for this purpose. However, they have the disadvantage of increasing the cost of the final product and contributing to the increase in antimicrobial resistance.

In addition, the use of antibiotics as growth-promoting food additives creates difficulties for the food industry in commercialization between countries, since animals whose food contains antibiotics are not allowed in some international markets. The European Union, for example, has banned the use of antibiotics as growth-promoting additives in 2006.

In order to overcome the disadvantages of using antibiotic compounds, the industry started to use additives from natural sources obtained from, for example, plant extracts and essential oils. However, such natural additives have cost and scalability problems, as they are products often obtained by costly and difficult mass production processes.

There are documents in the state of the art disclosing the use of natural products in food compositions for different purposes, which indicates a trend towards the use of materials from renewable sources, but without solving the technical challenge disclosed herein, which is to achieve an additive capable of improving the animal's zootechnical performance while being produced on a large scale and at low cost.

The document entitled "The Potential of Phytogenic Feed Additives in Pigs and Poultry", J.D. van der Klis and E. Vinyeta-Punti, describes the use of essential oils, aromatic herbs, and spices as food additives. Such additives work by increasing the palatability of diets, promoting enzymatic changes in the gastrointestinal tract, preventing infections, or even altering reproductive performance in addition to having an antioxidant effect. However, the document highlights that the antimicrobial activity of plant extracts is only evident at high concentrations of extract, so that synergistic effects with other food additives, such as organic acids or enzymes, need to be studied to reduce the minimum effective concentration.

US 2004/0241194 describes a combination of eucalyptus leaves, bilberry leaves, and fenugreek seeds extracts as a food supplement for inclusion in a food composition for the curative or preventive treatment of coccidiosis in ruminants, poultry, rabbits and pigs. In the food supplement, eucalyptus extract is present in the form of an essential oil which represents 8 to 15% by weight of said supplement. In addition to being effective in the curative or preventive treatment of coccidiosis, the supplement described therein proved to be beneficial to the weight gain of animals.

US 7,150,889 discloses the use of plant extracts from the *Eucalyptus* genus in food compositions aimed at decreasing weight gain and preventing diseases related to weight gain and fat accumulation. The purpose of the invention is to provide an anti-obesity agent. The document makes it clear that eucalyptus extract has an effective action in inhibiting weight gain.

The document entitled "Effects of Supplementation of Eucalyptus (E. Camaldulensis) Leaf Meal on Feed Intake and Rumen Fermentation Efficiency in Swamp Buffaloes" Thao et al., discloses that food supplementation with eucalyptus leaves improves the stability of fermentation in the buffalo rumen. Different types of essential oils can be extracted from eucalyptus leaves.

In this context, there is a need for additives from natural and renewable sources of low cost and capable of being obtained on a large scale. Additive studied in the art is lignin, a product from a natural and renewable source, of low cost and capable of being obtained on a large scale.

Historically, lignin was considered only as waste material, a byproduct of cellulose extraction. It is present in large quantities in all plants. Grasses contain 17 to 24% by wight of lignin, softwoods from 18 to 25% and hardwood from 27 to 33% by weight.

Its worldwide production capacity is estimated to be 50 million tons per year, but the vast majority of this quantity being used as a source of heat and energy in the production of cellulose. With the evolution of the market and new players therein, a growth of around 2.5% in 5 years in the global lignin market is estimated.

Due to its great availability and low cost, lignin has been used as an interesting alternative to eliminate the scalability problem experienced with the use of natural products. In this context, there are documents in the state of the art that already describe the application of lignin in the food industry.

DE 10106078 describes the use of lignin in food for human or animal consumption. In that document, the lignin present in food compositions acts as an adsorbent or binder for toxins and has the function of binding to mycotoxin and eliminating it in feces in order to prevent food poisoning.

US 2013/0164413 in turn describes the use of lignin itself as a source of fiber, or as a component of a source material of fiber, in feed for cats and dogs, especially cats. Although it was mentioned that lignin itself could be used as the source of fiber, the document relates only to crude lignin contained in grains and vegetables or present in the wood pulp. The purpose of this use is to provide a composition that helps in eliminating hairballs due to grooming formed in the animal's organism using wood components such as a dietary fiber containing lignin. In this document there is no use of lignin extracted from wood as a food additive but rather its addition as a functional fraction of dietary fiber. Apparently, there is no systemic absorption of the fiber source and consequently there is no improvement in the animal's zootechnical performance. In fact, there is an improvement in the formation of hairballs in the cat's gastrointestinal tract which is promoted by the addition of dietary fiber containing lignin, as it acts by stimulating the passage of the hairball through the digestive tract with its consequent elimination.

WO 2017/056275 also addresses the problem of hairballs formation due animal's grooming. According to this document, the formation of a hairball in the animal's body induces the vomiting of the hairball and consequently of the food eaten by the animal. As a solution to the problem of constant vomiting (on average every two days), publication WO 2017/056275 describes an animal feed comprising refined cellulose which comprises a lignin content of less than 1% by weight, more preferably 0, 3 5-wt or less. Again, there is no systemic absorption of the cellulose contained in the food by the animal and the source of lignin is crude lignin contained in the cellulose.

However, although the use of lignin in animal diets as a food additive has already been described, there is still a need in the art for food additives capable of improving zootechnical performance based on natural and renewable sources. This need was met by the food composition of the present invention which comprises up to 5% by weight of lignin or comprises an additive composition comprising up to 99% of lignin, wherein the lignin is preferably isolated lignin. Unlike what can be seen in the art, the lignin present in the compositions of the invention is absorbed and acts in the animal organism in a beneficial manner, even improving the characteristics of products originated from said animal.

SUMMARY OF THE INVENTION

Described herein is a food composition comprising up to 5% by weight of a food additive, wherein the food additive is lignin.

In a preferred embodiment, the composition comprises from 1 to 3% by weight of the food additive.% by weight

Also described herein is an additive composition comprising up to 99% by weight of lignin.

In one embodiment of the invention, the additive composition is a supplement, concentrate, premix, core, among others.

In a preferred embodiment of the invention, the additive composition is for preparing a food composition comprising up to 5% by weight of a food additive, wherein the food additive is lignin.

Also described herein is a food composition comprising said additive composition.

In one embodiment of the invention, the lignin present in the food and additive compositions of the invention is isolated lignin. In a preferred embodiment of the invention, the lignin is kraft lignin.

In a more preferred embodiment, the kraft lignin is hardwood kraft lignin.

In a preferred embodiment, the hardwood is eucalyptus wood.

The food compositions of the present invention comprise typical animal feed components such as sugar, flour, bran, starch, sugarcane juice, sugarcane molasses, yeast, powdered milk, oils, sorghum, corn, roasted whole soy, amino acids, limestone, dicalcium phosphate, salt, vitamins, folic acid, choline chloride, antioxidants, minerals, and other typical food composition components.

In a preferred embodiment, the food composition of the present invention comprises lignin as part or not of an additive composition, corn, soybean meal, dicalcium phosphate, limestone, soybean oil, salt, D-methionine, vitamins A, D3, E, K3, thiamine, riboflavin, pyridoxine, B12, biotin and niacin, calcium pantothenate, folic acid, choline chloride, antioxidant, iron, copper, manganese, zinc, iodine, and selenium.

In one embodiment of the invention, the food composition is an animal feed composition.

In one embodiment of the invention, the food composition improves the animal's zootechnical performance.

In a preferred embodiment, the improvement of zootechnical performance is selected from the group consisting of increased weight gain, reduced food consumption, reduced feed conversion, reduced fat levels, reduced fat deposition in the abdominal cavity, and histomorphological intestinal changes.

In a preferred embodiment, the reduction in fat levels is a reduction in the levels of LDL, HDL, triglycerides, plasma oxidation, or Tbars.

In a preferred embodiment, the intestinal histomorphological changes are an increase in crypts depth or an increase in villi height. In another preferred embodiment, intestinal histomorphological changes occur in the jejunum and/or duodenum.

In one embodiment of the invention, the food composition improves the characteristics of products of animal origin.

In a preferred embodiment, the improvement of characteristics in products of animal origin is selected from the group consisting of delaying the *postmortem* oxidative effects in chicken meat, reduced oxidation, and cholesterol in eggs, and increase in resistance and weight of eggshells.

In one embodiment of the invention, the food composition is for feeding livestock and companion animals.

In another preferred embodiment of the invention, the livestock is monogastric animals.

In a more preferred embodiment, the food composition is for feeding poultry.

Also described herein is the use of lignin as a food additive in a food composition or in an additive composition.

In one embodiment, described is the use of lignin as a food additive in a food composition, wherein said food composition comprises up to 5% by weight of lignin. More preferably, the food composition comprises from 1 to 3% by weight of lignin.

In one embodiment, lignin is kraft lignin, preferably hardwood kraft lignin, more preferably eucalyptus kraft lignin.

In one embodiment, the use of lignin as a food additive in a food composition is described, wherein said food composition is an animal feed composition.

In one embodiment, the aforementioned food composition improves the animal's zootechnical performance.

In one embodiment, the improvement of zootechnical performance is selected from the group consisting of increased weight gain, reduced food consumption, reduced feed conversion, reduced fat levels, reduced fat deposition in the abdominal cavity, and histomorphological intestinal changes.

In a preferred embodiment, the reduction in fat levels is a reduction in the levels of LDL, HDL, triglycerides, plasma oxidation, or Tbars.

In a preferred embodiment, the intestinal histomorphological changes are an increase in crypts depth or an increase in villi height. In another preferred embodiment, intestinal histomorphological changes occur in the jejunum and/or duodenum.

In one embodiment, the food composition improves the characteristics in products of animal origin.

In a preferred embodiment, the improvement of characteristics in products of animal origin is selected from the group consisting of delaying the *postmortem* oxidative effects in chicken meat, reduced oxidation, and cholesterol in eggs, and increase in resistance and weight of eggshells.

In one embodiment, said food composition is for feeding livestock or pets.

In another preferred embodiment of the invention, the livestock is monogastric animals.

In a more preferred embodiment, the food composition is poultry feed.

Also described herein is the use of lignin for preparing a food composition to improve the zootechnical performance of animals and/or the characteristics in products of animal origin.

In one embodiment, described is the use of lignin for preparing a food composition to improve the zootechnical performance of animals and/or the characteristics of products of animal origin, wherein said food composition comprises up to 5% by weight of lignin. More preferably, the food composition comprises from 1 to 3% by weight of lignin.

Also described herein is the use of lignin for preparing an additive composition comprising up to 99% lignin, as well as the use of the additive composition for preparing a food composition to improve the zootechnical performance of animals and/or the characteristics in products of animal origin, wherein said food composition comprises up to 5% by weight of lignin. More preferably, the food composition comprises from 1 to 3% by weight of lignin.

In a preferred embodiment, the lignin is kraft lignin, preferably hardwood kraft lignin, more preferably eucalyptus kraft lignin.

In one embodiment, the food composition is an animal feed composition.

In one embodiment, the improvement of zootechnical performance is selected from the group consisting of increased weight gain, reduced food consumption, reduced feed conversion, reduced fat levels, reduced fat deposition in the abdominal cavity, and histomorphological intestinal changes.

In a preferred embodiment, the reduction in fat levels is a reduction in the levels of LDL, HDL, triglycerides, plasma oxidation, or Tbars.

In a preferred embodiment, the intestinal histomorphological changes are an increase in crypts depth or an increase in villi height. In another preferred embodiment, intestinal histomorphological changes occur in the jejunum and/or duodenum.

In one embodiment, the improvement of characteristics in products of animal origin is selected from the group consisting of delaying *postmortem* oxidative effects in chicken meat, reduced oxidation, and cholesterol in eggs, and increase in resistance and weight of eggshells.

In one embodiment, said food composition is for feeding livestock or pets.

In another preferred embodiment of the invention, the livestock is monogastric animals.

In a more preferred embodiment, the food composition is poultry feed.

BRIEF DESCRIPTION OF THE DRAWINGS

Figure 01 shows the generic chemical structure assumed for lignin.

Figure 02 shows a feasibility graph of the chicken batch fed with different levels of lignin during four productive cycles.

Figure 03 shows a graph of the regression equation for feed consumption (g/bird/day) in relation to the level of lignin inclusion to the diet after four productive cycles.

Figure 04 shows a graph of the regression equation for egg production (%) in relation to the level of lignin inclusion to the diet after four productive cycles.

Figure 05 shows a graph of the regression equation for egg weight (g) in relation to the level of lignin inclusion to the diet after four productive cycles.

Figure 06 shows a graph of the regression equation for the percentage of soiled eggs found for birds fed different lignin levels in the diet during four productive cycles.

Figure 07 shows a graph of the regression equation for specific gravity of eggs in relation to the level of lignin inclusion to the diet after four productive cycles.

Figure 08 shows a graph of the regression equation for eggshell resistance in relation to the level of lignin inclusion to the diet after four productive cycles.

Figure 09 shows a graph of the regression equation for the percentage of eggshells in relation to the level of lignin inclusion to the diet after four productive cycles.

Figures 10 to 15 shows graphs respectively indicating the total cholesterol contents in egg yolk, the Tbars contents in egg yolk, the resistance of the eggshell, the percentage of eggshell, the percentage of egg yolk and the percentage of egg albumen versus the storage period, in which the eggs are from laying hens fed with different lignin inclusion levels and storage of up to 30 days.

DETAILED DESCRIPTION OF THE INVENTION

The present invention relates to food compositions comprising lignin or comprising an additive composition which in turn comprises lignin as a food additive leading to an improvement in the animal's zootechnical performance and/or an improvement in the characteristics in products of animal origin. The present invention also relates to additive compositions comprising lignin.

Food additives are defined as products used in animal nutrition for the purpose of improving the quality of the food or improving the performance and health of the animal. The additives may be technological, sensory, nutritional, zootechnical, or coccidiostatic and histomonostatic. Additives may be included in the food composition as part of an additive composition comprising other additives or as isolated components. When included in the food composition as part of an additive composition, it may be a concentrated composition mainly comprising an additive component or it may be a vitamin supplement.

The food compositions of the invention may comprise up to 5% by weight, preferably 1 to 3% by weight of lignin or an additive composition comprising lignin.

The term "additive composition" means any composition added to the final product comprising vitamins, nutrients, or other performance-enhancing components, wherein the final product is intended for feeding animals. Non-limiting examples of additive compositions are supplements, concentrates, premixes, cores, among others.

For example, the additive compositions of the invention may be concentrates comprising up to 99% by weight of lignin or vitamin supplements comprising up to 5% by weight of lignin.

In addition to lignin or the additive composition comprising lignin, the food compositions of the present invention further comprise usual components of animal feed such as, for example, sugar; flours, such as flours from carob, sweet potatoes, coconut, fish, blood, meat and bones, and feathers; bran, such as cottonseed, peanut, canola, soy, rice and wheat bran; starch; sugarcane juice; sugarcane molasses; yeast; powdered milk; oils, such as canola, corn and soybean oil; sorghum; corn; roasted whole soy; amino acids; limestone; dicalcium phosphate; salt; vitamins, such as vitamins A, D3, E, K3, thiamine, riboflavin, pyridoxine, B12, biotin and niacin; folic acid; choline chloride; antioxidants; minerals, such as iron, copper, manganese, zinc, iodine, and selenium; and other typical food composition components.

In a preferred embodiment, the food composition of the present invention comprises lignin as part of an additive composition or not, corn, soybean meal, dicalcium phosphate, limestone, soybean oil, salt, D-methionine, vitamins A, D3, E, K3, thiamine, riboflavin, pyridoxine, B12, biotin and niacin, calcium pantothenate, folic acid, choline chloride, antioxidant, iron, copper, manganese, zinc, iodine, and selenium.

Lignin can be used on a large scale and has low cost, being an advantageous option compared to natural products currently used with this function.

Any type of isolated lignin may be used in the compositions of the invention, with kraft lignin being preferred.

Even more preferably, the food and additive compositions of the invention comprise hardwood kraft lignin, preferably eucalyptus.

Lignin can be incorporated into the food composition of the invention in the form of powder, granules, pellets, liquid, or as part of an additive composition comprising other additives. In the additive composition of the invention, lignin may be incorporated in the form of powder, granules, pellets, or liquid in order to be incorporated into the food composition.

Lignin can be technically defined as an amorphous material derived from dehydrogenative reactions of three types of phenylpropanoids: trans-coniferyl (type-G), trans-synaphyl (type-S), and trans-p-coumaryl (type-H) alcohols, which can be connected in different ways by covalent bonds having no repetitive unit (a characteristic of polymers) but rather a complex arrangement of such precursor units generating macromolecules.

As all natural matter, lignin presents substantial differences in its composition, structure, and purity, which affect its properties and consequently its application potentials. Such variations depend on the botanical origin, as the ratio of the generating units (H/G/S) changes according to the type of plant. For example, said ratio is 0-5/95-100/0 in softwood, 0-8/25-50/46-75 in hardwood and 5-33/33-80/20-54 in grasses.

Furthermore, there is another variable: the process of extracting lignin, as it is impossible to isolate it without chemical changes to its structure. One of the main points affected by the extraction process is the molecular mass of the isolated lignin (also called technical lignin), which can be in a very wide range of 260 to 50,000,000 g/mol. The main processes for extracting lignin from lignocellulosic materials are soda, kraft, sulfite, and organosolv.

The lignin from biomass (raw matter) is in the form of lignocellulosic biomass, which is mainly composed of cellulose, hemicellulose, and lignin. The processes to isolate lignin from lignocellulosic biomass generate structural changes and divisions in the native lignin chain. Therefore, the (isolated) extracted lignin has different characteristics and effects to the application of the crude lignin contained in the lignocellulosic fiber. Preferably, the food and additive compositions of the present invention comprise isolated lignin.

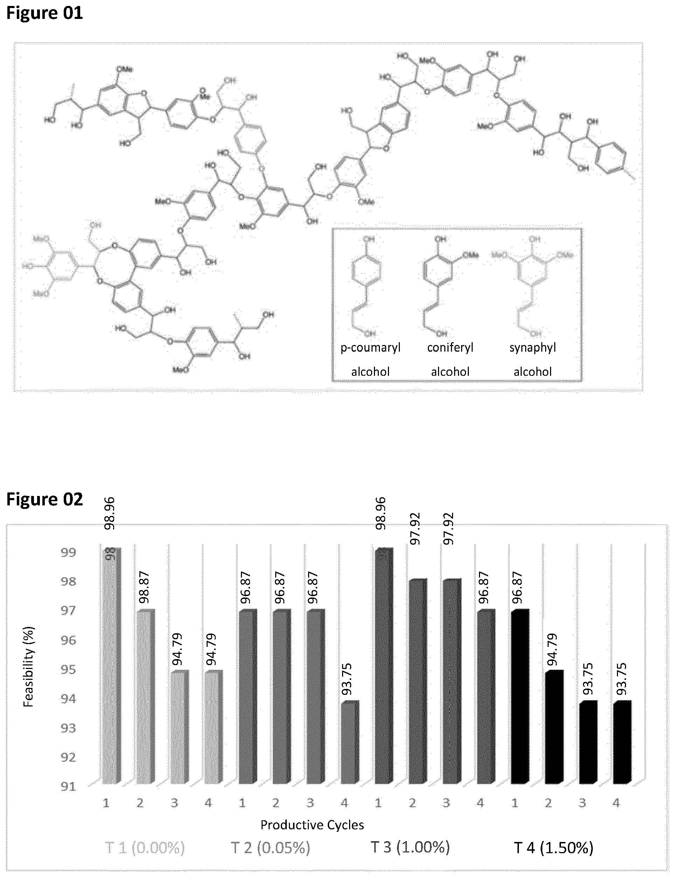

As can be seen, lignin has a very complex chemical structure. There are models aiming to describe it but there is no full definition. Figure 01 shows a supposed formula for such.

Kraft lignin is obtained by the kraft extraction process. Such a process is the most common in the pulp and paper industry. In it, wood particles are treated with a solution of sodium hydroxide and sodium sulfide at 150 — 180 °C. The process provides a lignin with a higher content of phenolic hydroxyl groups, stable carbon-carbon bonds in alkaline medium and introduction of quantities sulfur.

After the isolation by kraft process, the isolated lignin presents a fragmented and more reactive structure, which allows it to be absorbed by the animals' organism, whereas the crude lignin from biomass does not have this absorption capacity.

The food compositions of the invention comprising up to 5% by weight of lignin or an additive composition comprising up to 99% of lignin promote a prebiotic effect to the animal, providing an improvement to its health and zootechnical performance, in addition to optimizing the use of resources against a negative control diet. Furthermore, the compositions of the present invention promote an improvement in characteristics of products originating from the animals consuming said compositions.

Prebiotic is a nondigestible food component that has beneficial properties by selectively stimulating the growth or activity of microorganisms in the intestinal flora.

The improvement in zootechnical performance promoted by the food composition of the invention consists of increased weight gain, reduced food consumption, reduced feed conversion, increased live weight, reduced fat levels, reduced fat deposition in the abdominal cavity, promotion of intestinal mucosa development and/or intestinal histomorphological changes, when compared to a negative control diet.

A negative control diet according to the invention consists of a diet without lignin and/or any other food additives in its composition.

The expression "feed conversion" means a measure of animal productivity defined by the total feed consumption divided by the animal's weight gain.

The term "live weight" as used herein refers to the total weight of the live animal.

The term "reduction in fat levels" as used herein is understood as a reduction in serum contents of LDL, HDL, triglycerides, plasma oxidation or Tbars (substances reactive with thiobarbituric acid) and/or as a reduction in the levels of fat accumulation cavitary.

The term "LDL" (or "low-density lipoprotein") refers to a lipoprotein in the form wherein cholesterol is transported in the bloodstream to the cells and tissues of the body.

The term "HDL" (or "high density lipoprotein") refers to a lipoprotein in the form wherein cholesterol is transported in the bloodstream from tissues to the liver.

The term "lipoproteins" refers to macromolecules containing lipids and proteins known as apolipoproteins or apoproteins.

The term "triglycerides" refers to lipids that are esters formed by one molecule of glycerol and three molecules of one or more fatty acids.

The term "plasma oxidation" refers to lipid peroxidation.

The term "Tbars" refers to the quantification of lipid peroxidation and may be defined as substances that react to thiobarbituric acid.

The expression "development of the intestinal mucosa" means that there is an increase in the height and density of the intestinal villi, which are constantly renewed by the intestinal crypts.

The expression "intestinal histomorphological changes" means that there is an increase in crypts depth or in villi height of the gastrointestinal tract.

The term "villus" or "villi" refers to intestinal villi, which are structures that protect themselves in the intestinal lumen.

The term "crypts" refers to intestinal crypts, which are tubular glands found in the pits located between villi.

The improvement in the animal's health promoted by the food composition of the present invention consists of a reduction in serum levels of fat and/or plasma oxidation, the latter being provided by the antioxidant action of lignin.

The improvement in the animal's health may be understood as a reduction in the contents of LDL, HDL, triglycerides, plasma oxidation, or Tbars when compared to a negative control diet.

HDL contents higher than that of LDL are considered ideal as high-density lipoproteins collect cholesterol from cells and transport it back to the liver to be reprocessed and decreasing circulating cholesterol.

The food composition of the invention has the capacity to promote an 8 to 14% increase in weight gain and/or an 8 to 14% increase in live weight from.

The food composition of the invention has the capacity to also promote a 9 to 20% reduction in feed conversion of and/or a reduction in feed consumption of up to 16%.

The food composition of the invention also has the capacity to promote a 10 to 40% reduction in the accumulation of cavitary fat; a 2 to 24% reduction in blood LDL levels ; an 8 to 24% reduction in blood HDL levels; a 13 to 34% reduction in triglyceride levels and/or a 15 to 35% reduction in plasma oxidation.

The food composition of the invention has the capacity to promote an increase in villi height of up to 24% and/or a 10 to 40% increase in crypts depth.

These intestinal histomorphological changes can increase the nutrient absorption of animals, optimizing the use of food resources.

The food composition of the present invention promotes zootechnical performance similar to a positive control diet.

A positive control diet can be understood as a diet containing food additives typically used to improve zootechnical performance, such as, for example, antibiotics, natural extracts, or essential oils used as growth promoters.

In one embodiment of the invention, the food composition is an animal feed composition. Preferably, the feed composition is for feeding livestock and companion animals.

The expression "livestock" as used herein means an animal whose purpose of creation is obtaining meat, milk, eggs, wool, skin, leather, and honey, or any other product for commercial purposes. Non-limiting examples of livestock include cattle, chickens, fish, pigs, sheep, bees, crustaceans, rabbits, ducks, turkeys, ostriches, among others.

The expression "companion animals" as used herein means the animal belonging to the species created and maintained by man for entertainment, without the purpose of supplying products or by-products of economic interest. Non-limiting examples of pet animals include dogs, cats, fish, birds, turtles, among others.

Effects observed in the animal's organism, such as reduction of oxidative stress, can be extrapolated to the product obtained from such animal, as the product is part of or derived from the animal's organism. However, an improvement in zootechnical performance is not necessarily linked to obtaining an improved animal product.

Nonetheless, products obtained from livestock fed with the food composition of the present invention also have the antioxidant effect observed in the animals that have consumed it. Thus, such products have a lower oxidation level and speed, and therefore a longer durability/shelf life. Examples of such products are meat, milk, and eggs.

Due to the ingestion of the compositions of the present invention by animals, for example, a delay in *postmortem* oxidative effects on chicken meat, a reduction in oxidation and cholesterol in eggs, and an increase in resistance and weight of eggshells.

It was observed in the present invention that greater egg production by chickens fed with the compositions disclosed herein yielded thicker eggshells. This effect was surprising as it is expected that greater productions of eggs by birds yield thinner eggshells in such eggs. Such an unexpected effect represents a benefit, as thicker eggshells are less porous, which allows for better protection of gas exchange, generating less oxidation of the egg over time.

The food composition of the invention can be prepared by any conventional techniques used in the food or pharmaceutical industry and comprise additional components widely used in such industries. Similar to the food composition, the additive composition of the invention can be prepared by any conventional techniques used in the food or pharmaceutical industry and comprise additional food additives widely used in these industries.

The invention also relates to the use of lignin as a food additive in the food and additive compositions of the invention described above as a substitute for additives on the market.

Said use of lignin as a food additive gives the food compositions of the invention the same above described characteristics.

The invention also relates to the use of lignin as a food additive for the preparation of the above described food compositions or additives of the invention as a substitute for additives on the market.

The invention further relates to the use of the additive composition comprising up to 99% of lignin as a food additive for the preparation of the food compositions of the invention.

EXAMPLES

The examples presented herein are non-exhaustive, having the sole purpose of illustrating the invention, and should not be used as a basis for limiting it.

Examples herein are presented to demonstrate the benefits of the food composition of the present invention, both for animals and for products derived therefrom.

The study in example 1 evaluates the performance data of the animals. The parameters evaluated were body weight and body weight gain, feed intake, corrected feed conversion and mortality.

The study in example 2 assesses the carcass yield of animals fed with different food compositions.

Example 3 shows the evaluation of the hematological parameters of the animals in the study.

In example 4, the experiments carried out to evaluate intestine histomorphometry of animals fed with the food compositions of the invention are presented.

For the studies presented in examples 1 to 4, 600 male and female one-day-old male Paraíso Pedrêz lineage chicks from the same broiler breeders were used, all vaccinated against the Marek and Gumboro diseases and originating from the Aves do Paraíso (Itatiba-SP) hatchery. A completely randomized design (CRD) was used with four treatments and six replications of 25 birds each.

The results obtained in experiments 1 to 4 were set in tables and analyzed using analysis of variance (ANOVA) with the aid of the General Lineal Model (GLM) procedure of the statistical program SAS (2002) and the average between treatments were compared by the Tukey at 5% probability when significant. Mortality data were transformed to the root of $(x + 0.5)/100$, with x being the percentage of mortality (Steel and Torrie, 1980).

In examples 1 to 4, the control diet (CD) was formulated without the inclusion of ionophore anticoccidial and without any other additives and the other diets with isolated supplementation of lignin powder, as described below:

- Treatment 1: Control Diet (CD) + 1% lignin;
- Treatment 2: CD + 2% lignin;
- Treatment 3: CD + 3% lignin; and
- Treatment 4: Control Diet.

The composition of the experimental diets in such studies presented in examples 1 to 4 was based on corn, soybean meal, dicalcium phosphate, limestone, soybean oil, salt, DI-methionine, vitamins A, D3, E, K3, thiamine, riboflavin, pyridoxine, B12, biotin and niacin, calcium pantothenate, folic acid, choline chloride, antioxidant, iron, copper, manganese, zinc, iodine, and selenium in concentrations consistent with the percentage of nutrients required for the animal, according to the reference "ROSTAGNO, H.S. (Ed.). Tabelas brasileiras para aves e suínos: composição de alimentos e exigências nutricionais. 3rd ed. Viçosa: UFV, 2011". Lignin was included to replace the inert material present in the control diet, and all diets were isonutritive, formulated to meet the requirements for free range chicken in three phases: initial (1 to 21 days), growth (21 to 35 days), and final (35 to 63 days). Regardless of phase, all the feeds were manufacture on the same day and supplied in mash.

Example 5 represents a study divided into two experiments on the use of lignin in the feed of broilers. In the first experiment of the study, performance data and digestibility of ether extract, protein, and amino acids were evaluated. In the second experiment, performance data, carcass characteristics, bone mineral matter, intestinal morphometry, triglyceride levels and blood cholesterol and meat quality were evaluated.

Finally, example 6 shows a study on the use of lignin in diets for laying hens, indicating the performance in said hens and advantages in the characteristics of eggs produced by them.

The main effects aiming at increasing the shelf life of animal products can be seen in aging assessments, both of chicken meat and eggs. In this sense, one of the parameters evaluated in examples 5 and 6 was Tbars, which aims to quantify the products of lipid oxidation reactive with thiobarbituric acid.

Example 1

This study shows the performance evaluation of the animals that received food compositions with lignin and the control diet food composition.

Performance evaluations were carried out at the Polo Leste Paulista. The birds were housed in a brickwork shed at the APTA Regional do Leste Paulista, with dimensions of 23 m long and 9 m wide, equipped with 24 boxes of 1.75 x 1.65 m with capacity for 25 birds each. The boxes were equipped with individual electric hoods, independent feeders, and drinkers for each stage of bird growth. The shed was built in brickwork with an unpaved floor, fans, clay tile cover, brickwork side walls (0.5 m high), side screen and sliding plastic curtains. 24 experimental units were used. Each unit was lined with a new litter of wood shavings spread evenly at a height of approximately 10 cm.

Each experimental unit was equipped with a semi-automatic tubular baby chick feeder (metallic with plastic tray, 10 kg capacity), nipple type waterer, 250 watt infrared lamp used as a heating source; on the tenth day of poultry farming, the boxes were equipped with an adult tubular feeder (metallic with plastic tray, capacity 20 kg).

The birds were removed from the boxes at random, weighed in batches of 25 birds and distributed in the experimental units.

Until the age of 14 days, the temperature control inside the boxes was done through an electric bell jar keeping the temperature within the thermal comfort range of the birds. After this period and until the end of the poultry farming (14 to 63 days), the heating management of the shed was carried out according to the ambient temperature and the birds' behavior, with the climate being controlled by means of fans (positive pressure) and handling of curtains, which remained open during the day and closed in the event of rain or at night, if the temperature forecast was below 15 °C. The room temperature was measured daily using a hi-low thermometer.

The lighting program used was 23 hours of daylight and 1 hour of darkness (at the end of the day), supplementing natural light with artificial light.

Dead birds were removed daily, and the mortality was recorded through the number of the experimental unit, bird weight and day of occurrence. The birds and leftover feed were weighed weekly, from the day of housing until the end of the experimental period, at 63 days of farming. Scales with a capacity of 15.0 kg were used for initial weighing and for dead birds, and 150 kg scales for other weekly weighings. The feeder was cleaned by the outside of the boxes twice a day, and dead birds were removed with a grabber. The feed intake was made in specific buckets for each treatment following the order of treatments (starting with the control).

Water and feed were provided *ad libitum* throughout the farming period. The feeding program used consisted of three feeding stages: initial (1 to 21 days), growth (21 to 35 days), and final (35 to 63 days) phases. The feeds were offered at will in the form of mash. Water was provided at will throughout the experimental period.

Performance data was obtained and analyzed in the accumulated periods. The following data was collected and/or calculated.

Body weight and body weight gain: all birds in each experimental portion were weighed at the age of 1, 7, 14, 21, 28, 35, 42, 49, 56, and 63 days to calculate average body weight. The weight gain was calculated in accumulated periods through the difference between the weight of the birds at the beginning of each week and the weight of the birds at the end of the week.

Feed consumption: the feed consumption was determined by the difference between the amount of feed provided at the beginning and the leftovers remaining at the end of each weekly period, the obtained result was divided by the average number of birds in each experimental portion.

Corrected feed conversion: feed conversion was calculated by dividing the total weight of feed consumed by the birds in the portion, expressed in kilograms, by the total weight of the birds in the same period also expressed in kilograms. In the calculation, the weight and feed consumption of the dead birds in each evaluated period was considered.

Mortality: the mortality as well as the weight of dead birds were collected daily, and from this data the result given by the percentage of dead birds at the end of each period in relation to the initial number of birds housed was calculated.

The average performance results in the 1-21 days old farming period are shown in Table 1. There was no treatments-sex interaction.

Table 1: Performance of 21 days-old free range chickens submitted to diets with different levels of lignin inclusion

| %Lignin | AWG (kg) | FC | LW (kg) | Mort. | AFC (kg) |
|---------|----------|---------|---------|---------|----------|
| 0% | 0.3483b | 2.2783c | 393.02a | 0.02667 | 0.6583b |
| 1% | 0.3933a | 1.8683a | 433.49b | 0.02667 | 0.550a |
| 2% | 0.3883a | 1.9450ab | 429.91b | 0.04000 | 0.7783c |
| 3% | 0.3817a | 2.0850b | 425.57b | 0.04667 | 0.6933bc |
| **Average** | 0.3779 | 2.0440 | 420.50 | 0.035 | 0.670 |
| **CV\*** | 5.22 | 10.35 | 4.63 | 100.61 | 10.24 |

(continued)

| %Lignin | AWG (kg) | FC | LW (kg) | Mort. | AFC (kg) |
|---|---|---|---|---|---|
| Prob | 0.003 | 0.0164 | 0.065 | 0.6972 | 0.0001 |
| AWG = average weight gain; FC = feed conversion; LW = live weight; Mort = Mortality; AFC = average feed consumption; and a, b, c = averages for each factor followed by different letters in the line differ from each other by the Tukey's test (P <0.05).<br>*CV: coefficient of variation. | | | | | |

The effect of treatments on the average results of the variables average weight gain (AWG), feed conversion (FC), live weight (LW), and average feed consumption (AFC) was observed (Table 1). Diets containing lignin provided better results when compared to control diets in isolation. The birds that received 1% lignin consumed less feed, obtained better feed conversion, better average weight gain and higher live weight at the end of 21 days.

The average performance results in the 21-42 days old farming period are shown in Table 2.

Table 2: Performance of 21 to 42 days old free range chickens submitted to diets with different levels of lignin inclusion

| %Lignin | AWG (kg) | FC | LW (kg) | Mort. | AFC (kg) |
|---|---|---|---|---|---|
| 0% | 0.785 | 2.35 | 1.23 | 0.000 | 1.84 |
| 1% | 0.737 | 2.53 | 1.15 | 0.002 | 1.86 |
| 2% | 0.787 | 2.41 | 1.22 | 0.000 | 1.88 |
| 3% | 0.783 | 2.40 | 1.22 | 0.000 | 1.88 |
| Average | 0.773 | 2.42 | 1.20 | 0.067 | 1.86 |
| CV* | 5.55 | 13.05 | 4.26 | 244.95 | 12.39 |
| Prob | 0.16 | 0.79 | 0.62 | 0.022 | 0.98 |
| AWG = average weight gain; FC = feed conversion; LW = live weight; Mort = Mortality, and AFC = average feed consumption.<br>*CV: coefficient of variation. | | | | | |

Contrary to the results observed with the feed with additives in the initial poultry farming period, the inclusion of lignin in the birds' diet in the 21 to 42 days-old farming period did not change the researched performance variables. This happens because the animals have a deficient immune system in the first days of life and the food composition of the invention is more likely to assist in the defense of the immune system and consequently show visible differences in the variables. For the 21 to 42 days farming period, the birds' immune system was already consolidated and, therefore, the animals were not challenged - so there was no difference.

The average performance results in the 42-63 days old farming period are shown in Table 3. There was no treatments-sex interaction.

Table 3: Performance of 42 to 63 days old free range chickens submitted to diets with different levels of lignin inclusion

| %Lignin | AWG (kg) | FC | LW (kg) | Mort. | AFC (kg) |
|---|---|---|---|---|---|
| 0% | 0.760 | 3.213 | 1.9902 | 0.02833 | 2.4275 |
| 1% | 0.779 | 3.181 | 1.9138 | 0.04000 | 2.4487 |
| 2% | 0.794 | 3.119 | 2.000 | 0.02667 | 2.4735 |
| 3% | 0.777 | 3.267 | 1.9793 | 0.00667 | 2.5250 |
| Average | 0.777 | 3.196 | 1.9708 | 0.02542 | 2.4687 |
| CV* | 9.37 | 12.95 | 5.19 | 148 | 10.75 |

(continued)

| %Lignin | AWG (kg) | FC | LW (kg) | Mort. | AFC (kg) |
|---|---|---|---|---|---|
| Prob | 0.8821 | 0.9401 | 0.4733 | 0.5023 | 0.9284 |

AWG = average weight gain; FC = feed conversion; LW = live weight; Mort = Mortality, and AFC = average feed consumption.
*CV: coefficient of variation.

Regarding the average weight gain, the birds that received the feed with 1, 2 and 3% of lignin as additive had a greater weight gain compared to the control. The feed conversion was better in the group of birds that received the feed with 1 and 2% of lignin as additive and the live weight of the birds that received feed supplemented with 2% of lignin was higher than the other three studied groups.

The average performance results in the total 1-63 days old farming period are shown in Table 4. There was no treatments-sex interaction.

Table 4: Performance of 63 days-old free range chickens submitted to diets with different levels of lignin inclusion

| %Lignin | AWG (kg) | FC | LW (kg) | Mort. | AFC (kg) |
|---|---|---|---|---|---|
| 0% | 1.9338 | 2.555 | 1.9883 | 0.0550 | 4.922 |
| 1% | 1.8637 | 2.615 | 1.9150 | 0.0933 | 4.862 |
| 2% | 1.9655 | 2.615 | 2.000 | 0.0667 | 5.132 |
| 3% | 1.9408 | 2.633 | 1.9817 | 0.0533 | 5.100 |
| Average | 1.9259 | 2.605 | 1.9713 | 0.067 | 5.004 |
| CV* | 5.78 | 9.62 | 5.21 | 89.63 | 8.58 |
| Prob | 0.4475 | 0.9525 | 0.4920 | 0.6433 | 0.6417 |

AWG = average weight gain; FC = feed conversion; LW = live weight; Mort = Mortality, and AFC = average feed consumption.
*CV: coefficient of variation.

As explained above, this happens because the animals have a deficient immune system in the first days of life and the food composition of the invention is more likely to assist in the defense of the immune system and consequently show visible differences in the variables. Thus, the performance of the birds was better in the 21-day period. For the 21 to 63 days farming periods, the immune system of the birds is already consolidated, and the animals are therefore not challenged.

Example 2

This study shows the effects of the compositions of the present invention on the animals' carcass yield and vital organs.

The handling of the birds and the experiment site were as described in example 1.

Carcass yield evaluations were carried out at the PRDTA Leste Paulista. At 63 days of farming, 2 birds per experimental unit (one male and one female) were banded, a total of 12 birds/treatment. The birds were fasted for eight hours and transported in plastic cases to the procedure site, in which they were slaughtered by bleeding after electric stunning. The cuts obtained were eviscerated carcass (without feet, neck, and head), bone-in chest, legs (thigh + drumstick), back, and wings. The carcass yield calculation was based in the live weight on the platform immediately before slaughter and the weight of the carcass eviscerated and cooled, without head, neck and feet were. Yields of breast and legs (thigh and drumstick) were calculated in relation to the weight of the eviscerated carcass.

There was a significant treatment effect for the abdominal fat %, the other characteristics did not show significant effects for the treatments (Tables 5.1 and 5.2). Yields were calculated as the percentage relative to live weight ((part weight x 100)/live weight).

Tables 5.1 and 5.2: Carcass yield (%) and vital organs of 63 days old male (M) and female (F) free range chickens submitted to diets with different levels of lignin inclusion.

Table 5.1

| Lignin | LW | CarW | %CY | %WingY | %BreastY | %LegY |
|---|---|---|---|---|---|---|
| 0% | 2148 | 1443 | 66.96 | 14.38 | 26.14 | 31.69 |
| 1% | 2046 | 1397 | 68.10 | 14.62 | 26.62 | 32.20 |
| 2% | 2216 | 1492 | 67.43 | 14.41 | 26.78 | 31.98 |
| 3% | 2136 | 1435 | 67.22 | 14.78 | 26.07 | 31.99 |
| Sex | | | | | | |
| M | 2412a | 1627a | 67.32 | 14.58 | 25.49a | 32.62a |
| F | 1861b | 1257b | 67.54 | 14.51 | 27.31b | 31.31b |
| TREAT | 0.373 | 0.657 | 0.615 | 15.41 | 0.741 | 0.786 |
| SEX | 0.000 | 0.000 | 0.733 | 15.13 | 0.002 | 0.001 |
| TREAT*S* | 0.892 | 0.853 | 0.609 | 0.06 | 0.614 | 0.684 |
| CV** | 10.96 | 12.75 | 3.23 | 6.33 | 7.11 | 3.82 |
| Average | 2137 | 1442 | 67.43 | 14.54 | 26.40 | 31.96 |

LW = live weight; CarW = eviscerated carcass weight; CY = carcass yield; WingY = wing yield; BreastY = breast yield; LegY = leg yield, and a and b = averages for each factor followed by different letters in the line differ from each other by the Tukey's test (P <0.05).
*TRAT*S = treatment-sex interaction.
**CV: coefficient of variation.

Table 5.2

| Lignin | %BackY | %HeartY | %GizzardY | %LiverY | %ABfatY |
|---|---|---|---|---|---|
| 0% | 27.06 | 0.43 | 2.11 | 1.75 | 3.54b |
| 1% | 25.99 | 0.42 | 2.22 | 1.79 | 2.20a |
| 2% | 26.20 | 0.44 | 2.17 | 1.66 | 3.16ab |
| 3% | 26.20 | 0.43 | 2.21 | 1.80 | 3.02ab |
| Sex | | | | | |
| M | 26.63 | 0.46b | 2.01a | 1.70 | 2.81 |
| F | 26.10 | 0.40a | 2.34b | 1.80 | 3.14 |
| TREAT | 0.241 | 0.920 | 0.835 | 0.511 | 0.006 |
| SEX | 0.189 | 0.001 | 0.002 | 0.166 | 0.210 |
| TREAT*S* | 0.545 | 0.057 | 0.507 | 0.176 | 0.801 |
| CV** | 5.21 | 12.62 | 15.09 | 13.68 | 29.86 |
| Average | 26.36 | 0.43 | 2.18 | 1.75 | 2.98 |

BackY = back yield; HeartY = heart yield; GizzardY = gizzard yield; LiverY = liver yield; ABfatY = abdominal fat yield, and a and b = means for each factor followed by different letters in the line differ from each other by the Tukey's test (P <0.05).
*TRAT*S = treatment-sex interaction.
**CV: coefficient of variation.

Despite the higher (P <0.05) live weight at slaughter for males, the sex of the animals did not influence (P> 0.05) the observed value for carcass, heart, and wing yield. The results of cutting yield responded as expected, in which females showed higher results for gizzard, fat, liver, and breast yield, whereas males showed better back and legs yields, regardless of the action of lignin supplementation.

There was no significant effect of the treatment-sex interaction.

The results obtained demonstrate that the animals which received the feed with lignin as additive did not present a better carcass yield. However, these animals had a lower percentage of abdominal fat compared to animals which received the control diet. Among the animals that received the feed with lignin as additive, those that received 1% of lignin had a lower fat percentage. A lower accumulation of cavity fats is directly related to better performance, supporting the performance data obtained in the first 21 days of the experiment (example 1).

Example 3

This study shows the effects of the compositions of the present invention on the hematological parameters of the animals.

The management of the birds and the experiment site were as described in example 1.

At the time of bleeding during slaughter, the blood of 1 bird was removed per repetition, placed in heparinized tubes, centrifuged, and frozen for further analysis of Tbars, LDL, HDL, and triglycerides.

The results obtained for blood parameters are shown in Table 6.

Table 6: Blood parameters of 63 days old male (M) and female (F) free range chicken submitted to diets with different levels of lignin inclusion

| %Lignin | LDL | HDL | Tbars | Triglycerides |
|---|---|---|---|---|
| 0% | 73.43c | 117.63b | 35.1b | 127.22c |
| 1% | 57.19a | 107.10ab | 29.2a | 86.04a |
| 2% | 68.71b | 92.97a | 28.6a | 97.83ab |
| 3% | 71.36b | 92.11a | 23.5a | 109.11ab |
| SEX | | | | |
| M | 74.29a | 108.53a | 2.99 | 106.53 |
| F | 61.06b | 96.34b | 3.59 | 103.58 |
| TREAT | 0.2792 | 0.0078 | 0.000 | 0.000 |
| SEX | 0.04 | 0.0402 | 0.0579 | 0.5449 |
| T*S* | 0.546 | 0.0423 | 0.063 | 0.0617 |
| CV** | 32.24 | 19.40 | 32.34 | 15.94 |
| Average | 67.67 | 102.45 | 3.29 | 105.05 |
| a, b, and c = averages for each factor followed by different letters in the line differ from each other by the Tukey's test (P <0.05) *T*S: treatment-sex interaction **CV: coefficient of variation. | | | | |

The results obtained regarding the interaction of HDL with the treatment are shown in Table 7.

Table 7: Unfolding of interaction for HDL: treatment within given sex

| | SEX | |
|---|---|---|
| %Lignin | M | F |
| 1% | 114.58ab | 99.61 |
| 2% | 100.00a | 85.94 |
| 3% | 85.00a | 99.22 |

(continued)

| | SEX | |
|---|---|---|
| **%Lignin** | M | F |
| 0% | 134.56b | 100.71 |
| a and b = averages for each factor followed by different letters in the line differ from each other by the Tukey's test (P <0.05). | | |

The serum concentrations of triglycerides, Tbars, LDL cholesterol and HDL cholesterol were influenced (P> 0.05) by the treatments at age of 63 days (Table 6). Using the variation range of triglycerides (136 to 164) and LDL (72 to 130) described by González et al. (2001) for healthy chickens as a reference, the conclusion is that the values found can be considered normal regardless of treatment but the inclusion of lignin in the feed provided lower levels of LDL and HDL cholesterol, triglycerides, and Tbars in the birds' blood. The HDL and LDL cholesterol values presented were higher for males but generally indicate that both male and female chickens did not present harmful health conditions.

With regard to plasma oxidation (Tbars), treatments with a lower percentage of lignin had a higher concentration of malonaldehyde/mL of blood, proving in this case the antioxidant property of lignin that provided a reduction in the concentration of peroxides in the body. Nonetheless, there was no difference between treatments with the addition of lignin in terms of the amount of used residue.

Example 4

This study aimed to evaluate the intestinal histomorphometry of animals fed with the food compositions of the invention.

The management of the birds and the experiment site were as described in example 1.

Intestinal histomorphometry evaluations were performed at the Laboratório de Nutrição de Aves, FMVZ/UNESP, Botucatu campus, as described below.

At the time of slaughter, fragments of duodenum, Ileus, and jejunum were collected to make histology slides. Each intestinal segment corresponded to a slide. The villi height and crypts depth were determined by means of images, obtained under an optical microscope with 10X planapochromatic lens. The images were captured with a camera attached to the microscope and transferred to an image analyzer (Leica). Twenty readings of villi height and crypt depth were taken per slide. Villi height was measured from the apical region to the basal region, which corresponds to the upper portion of the crypts. The crypts were measured from the base to the transition region between the crypt and the villi.

The results of intestinal histomorphometry of 63 days old chickens are shown in Tables 8.1, 8.2, and 8.3.

Tables 8.1, 8.2, and 8.3: Villus height (VH), crypt depth (CD), and villus:crypt ratio (VC) of the small intestine segments of 63 days old broilers

Table 8.1

| | Jejunum | | |
|---|---|---|---|
| **%Lignin** | VH | CD | VC |
| 0% | 740.02a | 180.22a | 4.02 |
| 1% | 791.80ab | 199.89a | 4.61 |
| 2% | 909.37b | 209.85ab | 3.85 |
| 3% | 772.23ab | 253.05b | 4.09 |
| **SEX** | | | |
| M | 862.92b | 220.64 | 4.34 |
| F | 743.79a | 200.87 | 3.94 |
| **TREAT** | 0.0179 | 0.029 | 0.2579 |
| **CV** | 16.40 | 21.54 | 23.22 |

(continued)

| | Jejunum | | |
|---|---|---|---|
| %Lignin | VH | CD | VC |
| Average | 803.35 | 210.73 | 4.14 |
| a and b = averages for each factor followed by different letters in the line differ from each other by the Tukey's test (P <0.05) | | | |

Table 8.2

| | Ileus | | |
|---|---|---|---|
| %Lignin | VH | CD | VC |
| 0% | 538.29 | 145.55 | 3.85 |
| 1% | 537.58 | 146.81 | 4.04 |
| 2% | 560.58 | 156.77 | 3.93 |
| 3% | 548.70 | 149.21 | 3.90 |
| SEX | | | |
| M | 537.20 | 153.08 | 3.78 |
| F | 555.38 | 146.09 | 4.08 |
| TREAT | 0.9386 | 0.9037 | 0.9446 |
| CV | 18.62 | 26.80 | 19.46 |
| Average | 546.29 | 149.58 | 3.93 |

Table 8.3

| | Duodenum | | |
|---|---|---|---|
| %Lignin | VH | CD | VC |
| 0% | 993.99 | 250.55a | 4.02 |
| 1% | 992.97 | 275.10ab | 3.41 |
| 2% | 996.98 | 276.35ab | 3.77 |
| 3% | 1030.55 | 299.21b | 4.04 |
| SEX | | | |
| M | 964.78a | 288.21 | 3.74 |
| F | 1043.97b | 262.39 | 3.88 |
| TREAT | 0.8777 | 0.0596 | 0.1035 |
| CV | 12.73 | 15.27 | 17.90 |
| Average | 1004.38 | 276.30 | 3.81 |
| a and b = averages for each factor followed by different letters in the line differ from each other by the Tukey's test (P <0.05) | | | |

In the jejunum portions of the intestinal mucosa from the analyzed birds, the animals that received feed with lignin as additive had higher villus heights and crypt depths (P> 0.05), with males showing higher values than those observed for females. In the portions of the ileus and duodenum of the intestinal mucosa from the analyzed birds, the animals that received feed with lignin as additive had greater crypt depths (P>0.05), and villus height values were also higher for groups that received feed with 2 and 3% of lignin as additive.

These findings indicate greater nutrient absorption by birds.

There was no statistical evidence (P>0.05) of trophic effect of the treatments used at the ileus mucosa level, as well as treatment-sex interactions. There was no change in the villi:crypt ratio.

It is possible to observe that the addition of lignin to food compositions was beneficial, leading to improvements in the animals' performance and health in addition to the optimization of food resources. Thus, lignin is a substitute for performance-enhancing antibiotics and other types of zootechnical additives.

Example 5

The present study aims to evaluate the effect of lignin supplementation in the diet of 1 to 42 days old broilers.

The evaluations were carried out at the University of São Paulo (USP), School of Zootechnics and Food Engineering, Department of Zootechnics, Pirassununga campus.

The present study was divided into two experiments (hereinafter referred to as experiments I and II).

In experiment I, 490 male broiler chicks from the Cobb 500 lineage were used, housed in metabolic cages, which were distributed in a completely randomized design, with 5 treatments and 14 repetitions of 07 birds each, a total of 70 experimental portions. The 5 treatments performed are: Positive control diet with antibiotic 0% of lignin (T1); Negative control diet: no addition of lignin (T2); Negative control diet + 1% of lignin (T3); Negative control diet + 2% of lignin (T4); Negative control diet + 3% of lignin (T5).

The experimental diets were based on corn and soybean meal and are shown in Table 9:

Table 9: Experimental diets

|  | T1 | T2 | T3 | T4 | T5 |
|---|---|---|---|---|---|
| **Corn** | 49.61 | 49.61 | 49.61 | 49.61 | 49.61 |
| **Soybean meal** | 37.95 | 37.95 | 37.95 | 37.95 | 37.95 |
| **Soy oil** | 5.12 | 5.12 | 5.12 | 5.12 | 5.12 |
| **Inert** | 3.00 | 3.00 | 2.00 | 1.00 | 0.00 |
| **Bicalcium phosphate** | 1.87 | 1.87 | 1.87 | 1.87 | 1.87 |
| **Limestone** | 0.94 | 0.94 | 0.94 | 0.94 | 0.94 |
| **Salt** | 0.46 | 0.46 | 0.46 | 0.46 | 0.46 |
| **Methionine** | 0.40 | 0.40 | 0.40 | 0.40 | 0.40 |
| **Lysine** | 0.35 | 0.35 | 0.35 | 0.35 | 0.35 |
| **Threonine** | 0.15 | 0.15 | 0.15 | 0.15 | 0.15 |
| **Vitamins** | 0.10 | 0.10 | 0.10 | 0.10 | 0.10 |
| **Minerals** | 0.05 | 0.05 | 0.05 | 0.05 | 0.05 |
| **Lignin** | 0.00 | 0.00 | 1.00 | 2.00 | 3.00 |
| **Total** | 100.00 | 100.00 | 100.00 | 100.00 | 100.00 |
| **Calculated Nutritional Levels** | | | | | |
| **ME, kcal/kg** | 3,050 | 3,050 | 3,050 | 3,050 | 3,050 |
| **CP,%** | 22.20 | 22.20 | 22.20 | 22.20 | 22.20 |
| **Ca,%** | 0.96 | 0.96 | 0.96 | 0.96 | 0.96 |
| **DP, %** | 0.46 | 0.46 | 0.46 | 0.46 | 0.46 |
| **DLys, %** | 1.34 | 1.34 | 1.34 | 1.34 | 1.34 |
| **DSAA,%** | 0.98 | 0.98 | 0.98 | 0.98 | 0.98 |
| **DThr,%** | 0.87 | 0.87 | 0.87 | 0.87 | 0.87 |
| **Dval,%** | 0.89 | 0.89 | 0.89 | 0.89 | 0.89 |

(continued)

| Calculated Nutritional Levels | | | | | |
|---|---|---|---|---|---|
| CF,% | 3.21 | 3.21 | 3.21 | 3.21 | 3.21 |

The birds were fed with the experimental diets from the first day of age, and the collection of experimental excreta began on the 18th day for 3 days. After the collection period, the excreta were homogenized and frozen for storage. A 200 grams sample was taken from each repetition, which was dried in a forced ventilation oven, then the sample was ground and sent to the laboratory for bromatological analysis. The digestibility of the ether extract, protein and amino acids was calculated.

In experiment II, 1,300 male broiler chicks from the Cobb 500 lineage were used, housed in metabolic cages, which were distributed in a completely randomized design in experimental boxes, with 5 treatments and 20 repetitions of 13 birds each, a total of 100 experimental portions.

The treatments were the same as in the previous experiment, and the proposed lignin levels were achieved replacing the inert material present in the diets, taking place in the same manner as the positive control diet with the inclusion of 50g/ton. All treatments were sampled after the manufacture (200g each) and sent for laboratory analysis (dry matter, EE, crude protein, crude energy).

The birds were housed in boxes throughout the experimental period, with *ad libitum* offer of water and feed, and the lighting program adopted was 24 hours of light. The experimental diets are shown in Table 10:

Table 10: Experimental diets

| | Initial | Growth | Final |
|---|---|---|---|
| **Corn** | 49.61 | 53.27 | 56.42 |
| **Soybean meal** | 37.95 | 33.18 | 30.54 |
| **Soy oil** | 5.12 | 6.57 | 6.59 |
| **Inert** | 3.00 | 3.00 | 3.00 |
| **Dicalcium phosphate** | 1.87 | 1.50 | 1.11 |
| **Limestone** | 0.94 | 0.85 | 0.70 |
| **Salt** | 0.46 | 0.43 | 0.41 |
| **Methionine** | 0.40 | 0.38 | 0.35 |
| **Lysine** | 0.35 | 0.37 | 0.37 |
| **Threonine** | 0.15 | 0.16 | 0.20 |
| **Vitamins** | 0.10 | 0.10 | 0.10 |
| **Minerals** | 0.05 | 0.05 | 0.05 |
| **Lignin** | 0.00 | 0.00 | 0.00 |
| **Total** | 100.00 | 100.00 | 100.00 |
| **ME, kcal/kg** | 3,050 | 3,200 | 3,250 |
| **CP, %** | 22.20 | 20.62 | 19.54 |
| **Ca,%** | 0.96 | 0.82 | 0.66 |
| **DP, %** | 0.46 | 0.38 | 0.31 |
| **DLys, %** | 1.34 | 1.24 | 1.24 |
| **DSAA,%** | 0.98 | 0.91 | 0.91 |
| **DThr,%** | 0.87 | 0.82 | 0.76 |
| **Dval,%** | 0.89 | 0.95 | 0.93 |
| **CF,%** | 3.21 | 3.00 | 2.91 |

To promote health challenge, the birds were vaccinated against coccidiosis using 10 times the recommended dose to provoke an experimental challenge. Likewise, the birds were raised in a reused litter from a commercial farm, and the challenge was made via drinking water at the age of 11 days. For such, a solution was made with 1 kg of reused litter mixed with 4 liters

For bone ash analysis, nine 21 and 42 days old birds per treatment were submitted to the collection of the left tibia. The meat residues were removed and subsequently the left tibias were individually covered with gauze and dried for 12 hours in an oven at 105 °C. Then, the tibiae were placed in closed containers containing 90% ethyl ether and 10% methanol to be defatted. After two days, the bones were placed in a new solution of 90% ethyl ether and 10% methanol for 24 hours, dried in an oven at 105 °C, and weighed with the aid of a precision scale (0.0001g) for calculating the defatted dry matter. The bones were incinerated in the oven at a temperature of 600 °C for 24 hours to determine the ash in the defatted dry matter.

Regarding the animals' blood parameters, at 21 and 42 days, blood samples were collected from a bird with a near average weight (5% more or less than the average) of 8 portions per treatment through heparinized vacutainer tubes. The samples were centrifuged and frozen for further analysis of HDL, LDL, HLDL, triglycerides, and lipid oxidation (Tbars).

Regarding the animals' intestinal characteristics, at the age of 21 and 42 days, a bird with a near average weight (5% more or less than the average) of 8 parcels per treatment was slaughtered to collect the duodenum, jejunum, and ileus. The samples were collected, washed with distilled water, and placed into flasks containing Bouin for 24 hours for tissue fixation and further histological analysis. The evaluated morphometric characteristics were intestinal villi length, crypt depth, and villi/crypt ratio. Villi length was measured from the apical end of the villus to the junction of the villi with the crypt, and crypts depth was defined as the depth of invagination between the adjacent villi.

To determine performance and yield, birds and leftover feed were weighed at the age of 7, 21, 35, and 42 days to calculate average weight (kg), weight gain (kg), feed consumption (kg), and feed conversion (kg/kg). Mortality was recorded daily to correct feed intake and calculate feasibility (100 - mortality,%). Based on this information, the factor of production (FP = (WG (kg) x feasibility (%)/FC x age) x 100) was also calculated.

2 birds per repetition were slaughtered to estimate carcass yield and abdominal fat at the age of 42 days.

To assess meat quality, 2 breasts were collected from 5 portions of each treatment after slaughter, deboned, and skinned. Four 100 grams samples were taken from each breast, one used for cholesterol analysis and three for Tbars analysis. The samples were packed in individual expanded polyethylene trays, covered with transparent plastic film, and kept at a temperature between 1 and 3 degrees for 0, 7, and 14 days. At the end of each period, analyzes were performed.

For experiment I, the performance results of up to 21 days of the birds supplemented with different levels of lignin in the diet are shown in Table 11. No differences between treatments were observed for feed intake (P> 0.439). The birds which received the diet with the inclusion of 1% of lignin in the diet statistically had the same weight gain and the same feed conversion as the birds from the positive control treatment with the use of the performance-enhancing antibiotic (P<0.001). However, the inclusion of levels greater than 1% of lignin in the diet resulted in a decrease in the performance of the birds.

Table 11: Performance of broilers fed with lignin

| Treatments | Consumption, g | Gain | FC, g/g |
|---|---|---|---|
| **Positive control** | 1,132 | 895[a] | 1.266[a] |
| **Negative control - NC** | 1,106 | 837[b] | 1.322[b] |
| **NC + 1% of Lignin** | 1,101 | 878[a] | 1.275[a] |
| **NC + 2% of Lignin** | 1,093 | 807[c] | 1.357[c] |
| **NC + 3% of Lignin** | 1,105 | 831[b] | 1.331[b] |
| **No treatment** | 7 | 17 | 0.009 |
| **P,%** | 0.439 | <0.001 | <0.001 |

Regarding nutrient digestibility in experiment I, a positive effect was observed with the inclusion of 1% of fiber to the diet (see Table 12). Improvements of 1.65% in energy digestibility and of 1.5% in protein and amino acids digestibility were observed; results which likely contributed to the satisfactory performance of the birds in the age period of 1 to 21 days.

Table 12: Nutrient digestibility in broilers fed with lignin in the diet

| Treatments | Ethereal Extract | Protein | Amino acids |
|---|---|---|---|
| Positive control | 71.65[a] | 88.20[b] | 89.60[b] |
| Negative control - NC | 70.44[b] | 86.97[c] | 88.01[c] |
| NC + 1% of Lignin | 72.83[a] | 89.52[a] | 90.94[a] |
| NC + 2% of Lignin | 70.23 [b] | 87.10[c] | 88.23[c] |
| NC + 3% of Lignin | 70.30[b] | 87.18[c] | 88.15[c] |
| No treatment | 0.32 | 1.02 | 1.11 |
| P, % | 0.048 | 0.031 | 0.039 |

Regarding experiment II, performance, carcass, and digestive organs characteristics from birds fed different levels of lignin in the diet are shown in Tables 13 to 18 below.

Through the data obtained in experiment II, there were no statistical differences for final weight, weight gain, feed consumption, and feed conversion for birds fed with diets from different treatments observed in the period of 1 to 7 days (Table 13). Lower consumption and worse feed conversion were observed in birds in the negative control treatment (NC) when compared with the birds in the positive control treatment (PC) in the period of 1 to 21 days of farming (Table 14). However, birds fed with the inclusion of 2% lignin to the diet statistically had the same weight, same weight gain, and feed conversion as of birds fed with the PC birds. These results differ from those found in experiment I in that in the previous experiment the birds were raised in cages and without the presence of a health challenge as was observed in this research. In the period from 1 to 35 days (Table 15), it was observed that NC birds presented the worst final weight and weight gain. As demonstrated in the previous period, birds which received 2% of lignin in the diet had greater weight, greater weight gain, and better feed conversion than the other treatments. It can be seen in Table 16, which shows data from the complete experimental period (1 to 42 days), that the performance of birds fed with 2% lignin provided the best performance comparable with the performance of PC birds, thus confirming the results obtained in the previous periods. There were no statistical differences for carcass yield between birds fed with diets from different treatments. However, the inclusion of lignin to the diet decreased fat percentage (Table 17). Table 18 shows that the gizzard percentage was not affected by the treatments. However, the intestine percentage and length was higher in birds which received lignin in the diet when compared to birds from PC and NC treatments.

Table 13: Performance of broilers fed with lignin (1 to 7 days)

| Treatments | Initial weight g | Final weight, g | Gain, g | Consumption, g | FC, g/g |
|---|---|---|---|---|---|
| Positive control | 42 | 190 | 148 | 148 | 1.000 |
| Negative control - NC | 42 | 187 | 145 | 147 | 1.007 |
| NC + 1% of Lignin | 42 | 190 | 148 | 150 | 1.017 |
| NC + 2% of Lignin | 42 | 189 | 147 | 149 | 1.007 |
| NC + 3% of Lignin | 42 | 188 | 146 | 151 | 1.035 |
| No treatment | 0.05 | 0.71 | 0.70 | 1.07 | 0.007 |
| P, % | 0.968 | 0.652 | 0.664 | 0.710 | 0.563 |

Table 14: Performance of broilers fed with lignin (1 to 21 days)

| Treatments | Initial Weight g | Final weight, g | Gain, g | Consumption, g | FC, g/g |
|---|---|---|---|---|---|
| Positive control | 42 | 1,101[a] | 1,059[a] | 1,267[a] | 1.196[b] |
| Negative control - NC | 42 | 1,070[c] | 1,029[c] | 1,222[c] | 1.188[a] |
| NC + 1% of Lignin | 42 | 1,092[ab] | 1,050[ab] | 1,262[ab] | 1.202[c] |
| NC + 2% of Lignin | 42 | 1,094[a] | 1,052[a] | 1,259[b] | 1.197[b] |
| NC + 3% of Lignin | 42 | 1,074[c] | 1,032[c] | 1,264[ab] | 1.225[d] |

(continued)

| Treatments | Initial Weight g | Final weight, g | Gain, g | Consumption, g | FC, g/g |
|---|---|---|---|---|---|
| No treatment | 0.05 | 7 | 7 | 5 | 0.005 |
| P, % | 0.968 | 0.026 | 0.024 | 0.05 | 0.005 |

Table 15: Performance of broilers fed with lignin (1 to 35 days)

| Treatments | Initial Weight g | Final weight, g | Gain, g | Consumption, g | FC, g/g |
|---|---|---|---|---|---|
| Positive control | 42 | 2,382[a] | 2,340[a] | 3,263[a] | 1.394[d] |
| Negative control - NC | 42 | 2,312[c] | 2,270[c] | 3,096[e] | 1.364[bc] |
| NC + 1% of Lignin | 42 | 2,330[b] | 2,288[b] | 3,135[c] | 1.370[c] |
| NC + 2% of Lignin | 42 | 2,382[a] | 2,340[a] | 3,175[b] | 1.357[a] |
| NC + 3% of Lignin | 42 | 2,265[d] | 2,223[d] | 3,112[d] | 1.400[d] |
| No treatment | 0.05 | 11 | 12 | 19 | 0.006 |
| P, % | 0.968 | 0.026 | 0.024 | 0.042 | 0.002 |

Table 16: Performance of broilers fed with lignin (1 to 42 days)

| Treatments | Initial Weight g | Final weight, g | Gain, g | Consumption, g | FC, g/g |
|---|---|---|---|---|---|
| Positive control | 42 | 3,045[a] | 3,003[a] | 4,317 | 1.438[ab] |
| Negative control - NC | 42 | 2,954[b] | 2,912[b] | 4,205 | 1.444[b] |
| NC + 1% of Lignin | 42 | 2,965[b] | 2,923[b] | 4,318 | 1.477[c] |
| NC + 2% of Lignin | 42 | 3,035[a] | 2,993[a] | 4,288 | 1.433[a] |
| NC + 3% of Lignin | 42 | 2,904[c] | 2,862[c] | 4,295 | 1.501[d] |
| No treatment | 0.05 | 13 | 17 | 25 | 0.009 |
| P, % | 0.968 | 0.001 | 0.003 | 0.542 | <0.001 |

Table 17: Carcass characteristics of broilers fed with lignin (1 to 42 days)

| Treatments | Carcass Yield, % | Abdominal Fat, % |
|---|---|---|
| Positive control | 69.61 | 1.17[d] |
| Negative control - NC | 68.63 | 1.11[c] |
| NC + 1% of Lignin | 68.71 | 1.02[b] |
| NC + 2% of Lignin | 69.36 | 1.01[b] |
| NC + 3% of Lignin | 68.55 | 0.83[a] |
| No treatment | 0.15 | 0.02 |
| P, % | 0.066 | <0.001 |

Table 18: Digestive organs characteristics in broilers fed with lignin

|  | 21 days | | | 42 days | | |
|---|---|---|---|---|---|---|
| Treatments | Gizzard,% | Intestine, % | Intestine, cm | Gizzard, % | Intestine, % | Intestine, m |
| Positive Control | 2.39 | 9.37d | 1.84c | 1.62 | 5.93e | 2.33e |
| Negative control - NC | 2.37 | 9.96c | 1.84c | 1.62 | 6.24d | 2.43d |
| NC + 1% of Lignin | 2.2 | 10.29b | 2.05a | 1.67 | 7.13b | 2.59b |
| NC + 2% of Lignin | 2.44 | 10.17b | 2.06a | 1.66 | 6.78c | 2.53c |
| NC + 3% of Lignin | 2.19 | 10.52a | 2.01b | 1.54 | 7.31a | 2.65a |
| No treatment | 0.03 | 0.12 | 0.03 | 0.04 | 0.15 | 0.03 |
| P, % | 0.161 | 0.05 | 0.012 | 0.814 | 0.008 | 0.012 |

Furthermore, it was observed that none of the evaluated characteristics was influenced by treatment at the age of 21 days when blood parameters - indicated in Table 19 below - are observed. The absence of statistical differences at this age may have occurred due to the period of proving lignin to the animals' diet no being long enough to promote differences between treatments. However, when observing these same characteristics at the age of 42 days, a reduction in blood cholesterol, HDL and LDL levels is found when lignin is added to the diet of broilers with no variation for the levels of triglycerides and VLDL.

Table 19: Blood parameters of broilers fed with lignin in the diet

| Treatments | Triglycerides | Cholester ol | HDL | LDL | VLDL |
|---|---|---|---|---|---|
|  | mg/dL | mg/dL | mg/dL | mg/dL | mg/dL |
| 21 days |  |  |  |  |  |
| Positive control | 80.91 | 93.04 | 64.69 | 15.78 | 16.25 |
| Negative Control —NC | 73.11 | 90.48 | 59.00 | 14.60 | 14.71 |
| NC + 1% of Lignin | 69.70 | 90.46 | 61.30 | 16.67 | 14.00 |
| NC + 2% of Lignin | 59.86 | 97.08 | 66.93 | 18.15 | 12.00 |
| NC + 3% of Lignin | 85.05 | 93.30 | 61.31 | 14.93 | 17.00 |
| No treatment | 3.90 | 1.59 | 1.24 | 1.16 | 0.77 |
| P, % | 0.267 | 0.675 | 0.269 | 0.885 | 0.262 |
| 42 days |  |  |  |  |  |
| Positive control | 68.69 | 111.91b | 72.26a | 25.90a | 13.75 |
| Negative Control —NC | 93.77 | 114.69a | 66.68b | 22.38b | 18.86 |
| NC + 1% of Lignin | 88.38 | 94.27c | 62.98d | 17.59d | 17.75 |
| NC + 2% of Lignin | 93.61 | 95.96c | 64.34c | 12.69e | 18.75 |
| NC + 3% of Lignin | 67.6 | 92.13d | 60.07e | 18.77c | 13.29 |
| No treatment | 4.2 | 2.17 | 1.23 | 1.41 | 0.84 |
| P, % | 0.09 | <0.001 | 0.011 | 0.026 | 0.068 |

Regarding Tbars index (Table 20 below), an increase in the index was observed with the increase in the storage time. However, within the same storage date, the use of lignin in the diet increased the values of this parameter.

Table 20: Breast Tars index of broilers fed with lignin

| Treatments | 0 d | 7 d | 14 d |
|---|---|---|---|
| Positive control | 0.54d | 1.27b | 1.39c |
| Negative control - NC | 0.44e | 1.12c | 1.14d |
| NC + 1% of Lignin | 0.72b | 1.58a | 2.05a |
| NC + 2% of Lignin | 0.63c | 1.04d | 1.14d |
| NC + 3% of Lignin | 0.85a | 1.53a | 1.58b |
| No treatment | 0.03 | 0.06 | 0.07 |
| P, % | <0.001 | 0.022 | 0.001 |

As can be seen in Table 21, there were no statistical differences in the birds' mineral matter from the different evaluated treatments:

Table 21: Bone mineral matter (%) of broilers fed with lignin levels

| Treatments | 21 days | 42 days |
|---|---|---|
| Positive control | 42.64 | 41.79 |
| Negative control - NC | 40.32 | 43.12 |
| NC + 1% of Lignin | 39.58 | 43.91 |
| NC + 2% of Lignin | 38.9 | 42.05 |
| NC + 3% of Lignin | 40.02 | 42.16 |
| No treatment | 0.49 | 0.33 |
| P, % | 0.151 | 0.205 |

No statistical differences were observed for the intestinal morphology of the duodenum and jejunum at the age of 21 days by evaluating the intestinal morphometry data shown in Table 22 below. However, the use of 3% lignin in the diet decreased the VH:CD ratio in the birds' ileus. A greater intestinal villi length in relation to crypt depth is correlated with the intestinal health improvement, providing better uniformity and integrity of the mucosa in addition to providing greater nutrient absorption due to the increase in the absorption surface. At the age of 42 days, the use of lignin decreased the VH:CD ratio in the duodenum. However, the deepest crypt depth was observed in birds that received 2% of lignin in the diet for jejunum and Ileus and 1% for duodenum.

Table 22: Villus height (VH, mm), crypt depth (CP, mm), and their ratio (AV: PC) of broilers' intestines fed with lignin

| | 21 days | | | 42 days | | |
|---|---|---|---|---|---|---|
| Treatments | VH | CD | VH:CD | VH | CD | VH:CD |
| Duodenum | | | | | | |
| Positive control | 1,201 | 213 | 5.67 | 1,450 | 218 | 6.88[a] |
| Negative Control - CN | -1,223 | 230 | 5.61 | 1,380 | 219 | 6.38[b] |
| NC + 1% of Lignin | 1,170 | 210 | 5.6 | 1,374 | 257 | 5.38[d] |
| NC + 2% of Lignin | 1,248 | 221 | 5.8 | 1,287 | 241 | 5.36[d] |
| NC + 3% of Lignin | 1,205 | 180 | 6.72 | 1,296 | 223 | 5.82[c] |
| No treatment | 19.1 | 6.75 | 0.178 | 22.9 | 5.45 | 0.157 |
| P, % | 0.765 | 0.234 | 0.276 | 0.133 | 0.098 | 0.003 |
| Jejunum | | | | | | |
| Positive control | 874 | 171 | 5.17 | 963 | 163 | 6.038 |

(continued)

| | 21 days | | | 42 days | | |
|---|---|---|---|---|---|---|
| Treatments | VH | CD | VH:CD | VH | CD | VH:CD |
| **Duodenum** | | | | | | |
| **Negative Control - CN** | 904 | 189 | 4.92 | 911 | 172 | 5.33 |
| **NC + 1% of Lignin** | 924 | 170 | 5.43 | 883 | 166 | 5.44 |
| **NC + 2% of Lignin** | 913 | 163 | 5.70 | 926 | 169 | 5.52 |
| **NC + 3% of Lignin** | 994 | 182 | 5.52 | 890 | 154 | 5.81 |
| **No treatment** | 23.8 | 5.07 | 0.13 | 17.10 | 4.01 | 0.120 |
| **P, %** | 0.613 | 0.519 | 0.312 | 0.616 | 0.667 | 0.332 |
| **Ileus** | | | | | | |
| **Positive control** | 552 | 123 | 4.52 | 617 | 104[c] | 5.93[c] |
| **Negative Control - CN** | 527 | 118 | 4.48 | 685 | 105[c] | 6.55[a] |
| **NC + 1% of Lignin** | 561 | 124 | 4.55 | 684 | 110[b] | 6.20[b] |
| **NC + 2% of Lignin** | 521 | 109 | 4.86 | 600 | 126[a] | 4.82[d] |
| **NC + 3% of Lignin** | 482 | 127 | 3.81 | 651 | 108[b] | 6.03[c] |
| **No treatment** | 12.8 | 2.33 | 0.10 | 12.9 | 2.44 | 0.138 |
| **P, %** | 0.333 | 0.132 | 0.015 | 0.123 | 0.019 | 0.001 |

From the present study (data from experiments I and II), it was possible to conclude that there is a great potential for the use of lignin in broiler with improved performance and ingredient digestibility.

Due to the performance improvement and the conditions in which the study was conducted, the use of 2% of lignin in the diet of broilers was observed as a tool which can be used to replace the use of performance-enhancing antibiotic in poultry production.

Another great piece of information found in this study was the cholesterol and LDL reduction in the animals' bloodstream.

Example 6

The present study aims to evaluate the use of lignin in diets for laying hens. As will be shown in detail below, the study indicates both a performance improvement in the birds and advantages in the egg characteristics obtained by them.

The evaluations were carried out at the São Paulo State University (UNESP), School of Veterinary Medicine and Animal Science (FMVZ), Department of Animal Production, Botucatu campus.

For the evaluation, laying hens were housed in a conventional system, in a Californian type aviary, with metal cages 1.00 m long x 0.45 m deep x 0.40 m high, divided into 2 compartments, in which 4 birds were housed per compartment, a total of 8 birds per cage. The cages were individually equipped with metal feeders and nipple type waterer. 384 birds of the H&N Nick Chick lineage were used for this experiment.

The 384 birds in the present study received different treatments, which are classified as follows:

T1 - Diet with no lignin (96 birds);
T2 - Diet with 0.5% of lignin (96 birds);
T3 - Diet with of 1.0% of lignin (96 birds); and
T4 - Diet with 1.5% of lignin (96 birds).

The experimental period was subdivided into 4 cycles of 21 days, a total of 84 experimental days, when the birds were between 59 weeks and 71 weeks old. In the last three days of each experimental period, 72 eggs were collected per treatment, a total of 288 eggs per 21-day cycle. These eggs were used to evaluate the egg weight, specific gravity, yolk percentage, albumen percentage, and eggshell percentage and resistance.

The quality characteristics of the intestinal mucosa were also evaluated in order to verify possible changes in the absorptive capacity. For such, portions of the small intestine, segments of the duodenum, jejunum, and ileus were collected to assess the villus height, crypt depth and villus x crypt ratio.

Thus, 48 birds (12 per treatment) were euthanized at the beginning of the experiment and 48 birds (12 birds per treatment) at the end of the experiment, with 288 histological slides being made. At the beginning of the experimental period, blood and eggs were also collected from 48 birds for analysis of HDL (high density lipoprotein) and LDL (low density lipoprotein), total cholesterol, and Tbars (thiobarbituric acid reactive substances - nmol MDA/mL). At the end of the experiment, this evaluation was repeated for comparison purposes using 12 birds per treatment.

Eggs were collected at the end of the last experimental cycle and stored for up to 30 days for quality assessments called shelf life. For this evaluation, the quality of the eggs was verified every 10 days by means of specific gravity, eggshell resistance, eggshell, albumen and yolk percentages, total cholesterol, and Tbars, so that improvement in egg quality from inclusion of lignin to the birds' diet could be evaluated.

At the end of each experimental cycle, the animals' productive performance was evaluated by means of the following analyzes:

- Feed consumption (g/bird/day): whenever the feed was distributed to the birds, they were weighed and, at the end of each week, the surplus was weighed in the feeders, thus calculating the weekly feed consumption.
- Egg production (%): the eggs were collected every day and the production recorded in a specific file. Thus, it was possible to calculate the percentage of eggs produced in the 21-day period in relation to the number of birds housed in the experiment.
- Egg weight (g): the eggs collected daily were weighed in trays of 30 eggs minus the weight of the tray. Thus, it was possible to calculate the average weight of the eggs produced in each 21-day period.
- Feasibility (%): bird mortality was noted whenever it occurred. Therefore, it was possible to calculate the feasibility of the batch for a 21 day period (100 - mortality = feasibility).
- Feed conversion: once the egg weight, the number of eggs produced, and the feed consumption by the birds were obtained, it was possible to calculate the feed conversion per kilogram of feed consumed and per dozen eggs produced.

The quality of the eggs obtained by the birds of the present experiment was also evaluated using the following criteria:

- Specific gravity: for this evaluation, five saline solutions were prepared with densities of 1.060, 1.070, 1.080, 1.090 and 1.100 and placed in ascending order in identified containers. Firstly, the eggs were placed in the 1.060 container, and so on, until the eggs floated in the solution. The egg's specific gravity was represented by the lower density solution in which it emerged.
- Eggshell resistance to breakage (kgf): this evaluation was carried out using the TA.XT plus Texture Analyzer texturometer equipped with a 75 mm rupture probe (P/75), calibrated for a test speed of 1 mm/second of descent. The device marks the force required to break the eggshell, which was expressed in $kgf/cm^2$.
- Yolk and albumen percentage: yolk percentage was obtained considering the total egg weight and the yolk weight and the same measurement was performed for albumen according to the equations below:

$$\%G = \frac{Pyolk}{Pegg} \text{x } 100$$

$$\%\,Alb = \frac{Palbumen}{Pegg} \text{x } 100$$

- Eggshell percentage: the eggshell percentage was obtained considering the total egg weight and the eggshell weight, which was measured after the eggshells were dried in an oven at 60 °C for 3 days. The equation below expresses such measurement:

$$\%C = \frac{Pshell}{Pegg} \text{x } 100$$

- Soiled eggs (%): every day during egg harvesting, eggs with dirt in the eggshell were noted. Thus, it was possible to estimate the percentage of soiled eggs in each treatment.

The data from the above indicated analyzes obtained in the present study were submitted to the statistical package SAS 9.2 for Analysis of Variance, and the averages compared by the Tukey's Test ($p < 0.05$). The egg production and quality data were also submitted to regression evaluation ($p < 0.05$), in order to estimate the best level of lignin inclusion.

Figure 02 shows a graph illustrating the feasibility data for treatments T1 to T4 of the present study in each production cycle. As can be seen in the figure, the different levels of lignin inclusion did not affect ($p > 0.05$) the feasibility of the batch of birds, with each 1.04% representing one bird.

In addition, Table 23 below indicates the performance data of the birds studied, as well as the product characteristic referring to the % of soiled eggs.

Table 23: Performance of birds supplemented with different levels of lignin.

| Treatment | Cycle | Feed consumption (g/bird/day) | % Production | % Soiled Eggs | Weight of egg | FC (kg/dz) | FC (kg of feed/kg of egg) |
|---|---|---|---|---|---|---|---|
| T1 | 1 | 124.48 | 89.75 | 2.63 | 66.84 | 1.66 | 2.08 |
| | 2 | 123.79 | 89.55 | 1.03 | 64.94 | 1.66 | 2.13 |
| | 3 | 124.19 | 86.13 | 1.24 | 66.97 | 1.73 | 2.15 |
| | 4 | 127.22 | 89.89 | 1.28 | 68.56 | 1.70 | 2.06 |
| | Average | **124.92 B** | **88.83 B** | **1.55 B** | **66.83** | **1.69 B** | **2.10** |
| T2 | 1 | 130.00 | 89.04 | 4.47 | 67.29 | 1.75 | 2.17 |
| | 2 | 134.59 | 93.37 | 2.39 | 65.80 | 1.73 | 2.19 |
| | 3 | 130.63 | 88.49 | 1.11 | 67.64 | 1.77 | 2.18 |
| | 4 | 132.49 | 89.25 | 0.79 | 68.29 | 1.78 | 2.17 |
| | Average | **131.93 A** | **90.04 AB** | **2.19 A** | **67.26** | **1.76 A** | **2.18** |
| T3 | 1 | 131.47 | 90.91 | 1.37 | 67.08 | 1.74 | 2.16 |
| | 2 | 130.28 | 94.13 | 0.17 | 66.13 | 1.66 | 2.09 |
| | 3 | 127.35 | 89.64 | 0.38 | 67.62 | 1.70 | 2.10 |
| | 4 | 127.27 | 90.91 | 0.17 | 67.56 | 1.68 | 2.07 |
| | Average | **129.09 AB** | **91.40 A** | **0.52 C** | **67.10** | **1.69 B** | **2.11** |
| T4 | 1 | 128.01 | 90.49 | 3.56 | 66.82 | 1.70 | 2.12 |
| | 2 | 130.16 | 93.78 | 1.28 | 66.09 | 1.67 | 2.10 |
| | 3 | 127.49 | 89.59 | 1.23 | 66.78 | 1.71 | 2.13 |
| | 4 | 127.14 | 90.78 | 0.88 | 67.93 | 1.68 | 2.06 |
| | Average | **128.20 AB** | **91.16 A** | **1.74 B** | **66.91** | **1.69 B** | **2.10** |
| Coefficient of Variation (%) | | 5.31 | 2.44 | 6.78 | 4.08 | 2.11 | 4.67 |
| P-value | | 0.022 | 0.013 | 0.011 | 0.541 | 0.031 | 0.074 |

Averages followed by different letters in the column differ from each other using the Tukey's test ($p < 0.05$).

From the table above, a difference ($p < 0.05$) was found among treatments for several characteristics, such as feed consumption, egg production, soiled eggs percentage, and feed conversion for a dozen eggs produced at the end of four productive cycles.

In general, the best results were found for birds that received feed with 1% of lignin (T3). It was not possible to detect a difference ($p > 0.05$) for feed conversion per kilogram of eggs produced. It can be inferred that this characteristic was not different among the treatments tested, as feed consumption and the total weight of eggs produced are considered, which indirectly considers the batch's feasibility. As there was no difference for egg weight and feasibility characteristics, feed conversion per kilogram of eggs produced was not affected.

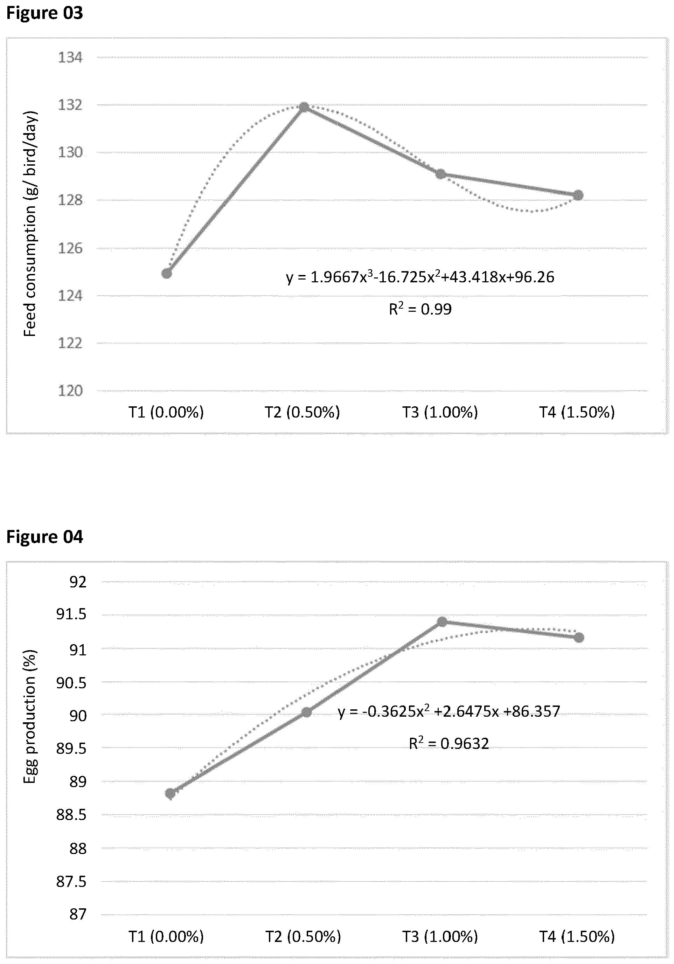

After regression evaluation for the birds' performance characteristics, a significant cubic regression for the characteristic of feed consumption and quadratic regression for egg production and egg weight were found, as can be seen in the graphs of figures 03 to 05. Through these figures, these characteristics were found to have higher values with inclusions between 0.50 and 1.00% of lignin.

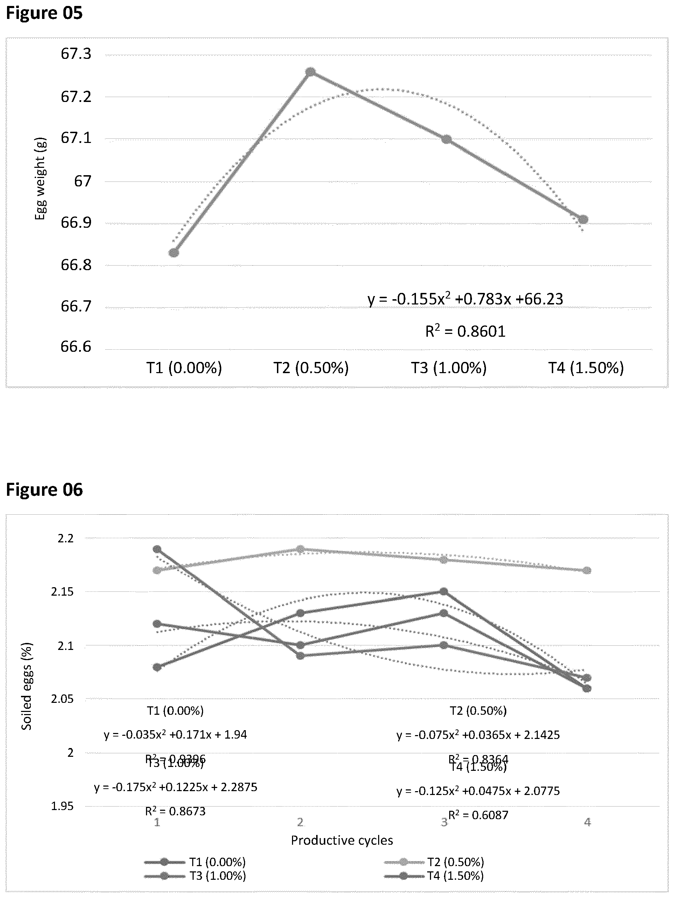

The regression evaluation for soiled eggs percentage can in turn be verified through the graph in figure 06. This evaluation demonstrated that there is a decreasing tendency between the third and fourth productive cycles, regardless of the treatment used. However, it cannot be said whether this behavior would continue for other subsequent cycles.

Finally, the egg quality of birds submitted to diets with different levels of lignin inclusion in the present study is shown in Table 24 below.

Table 24: Egg quality of birds supplemented with different levels of lignin.

| Treatment | Cycle | Egg weight (g) | Specific Gravity | Eggshell resistance (kgf) | % Yolk | Eggshell | % Albumen |
|---|---|---|---|---|---|---|---|
| **T1** | 1 | 67.11 | 1.08 | 3.86 | 27.04 | 9.26 | 63.7 |
| | 2 | 65.34 | 1.08 | 3.56 | 26.93 | 8.84 | 64.24 |
| | 3 | 69.44 | 1.08 | 3.65 | 25.79 | 8.75 | 65.46 |
| | 4 | 68.45 | 1.08 | 3.63 | 26.63 | 8.45 | 64.92 |
| | **Average** | **67.59** | **1.08 B** | **3.68 B** | **26.60** | **8.83 B** | **64.58** |
| **T2** | 1 | 66.56 | 1.09 | 3.95 | 26.2 | 9.28 | 64.51 |
| | 2 | 67.80 | 1.09 | 3.93 | 25.96 | 10.37 | 63.66 |
| | 3 | 69.07 | 1.08 | 3.85 | 26.39 | 8.77 | 64.84 |
| | 4 | 68.57 | 1.08 | 3.68 | 26.28 | 8.82 | 64.9 |
| | **Average** | **68.00** | **1.09 A** | **3.85 A** | **26.21** | **9.31 A** | **64.48** |
| T3 | 1 | 67.33 | 1.08 | 3.71 | 26.75 | 9.18 | 64.08 |
| | 2 | 67.16 | 1.10 | 3.82 | 26.30 | 9.04 | 64.66 |
| | 3 | 68.91 | 1.08 | 3.89 | 26.13 | 9.78 | 64.09 |
| | 4 | 68.40 | 1.08 | 3.74 | 26.59 | 8.91 | 64.5 |
| | **Average** | **67.95** | **1.09 A** | **3.79 A** | **26.44** | **9.23 A** | **64.33** |
| **T4** | 1 | 67.33 | 1.09 | 3.88 | 26.66 | 9.24 | 64.09 |
| | 2 | 68.85 | 1.09 | 3.72 | 26.43 | 8.88 | 64.69 |
| | 3 | 69.66 | 1.09 | 3.62 | 26.19 | 8.75 | 65.06 |
| | 4 | 68.33 | 1.08 | 3.59 | 26.74 | 8.71 | 64.55 |
| | **Average** | **68.54** | **1.09 A** | **3.70 B** | **26.51** | **8.90 B** | **64.60** |
| **Coefficient of Variation (%)** | | 6.87 | 2.31 | 13.01 | 4.67 | 5.09 | 4.85 |
| **P-value** | | 0.789 | 0.034 | 0.020 | 0.094 | 0.013 | 0.192 |

Averages followed by different letters in the column differ from each other using the Tukey's test (p <0.05).

As can be seen from the data in the table above for egg quality, the inclusion of lignin was found to be very efficient in improving eggshell quality, especially at the levels of 0.50 and 1.00% (T2 and T3, respectively). It should be noted that the specific gravity characteristics, eggshell resistance, and percentage of eggshell were better (p <0.05) for the eggs of birds which received these levels of inclusion of lignin to the diet. For specific gravity, treatment with inclusion of 1.50% of lignin also provided good results. The other characteristics were not influenced by the treatments.

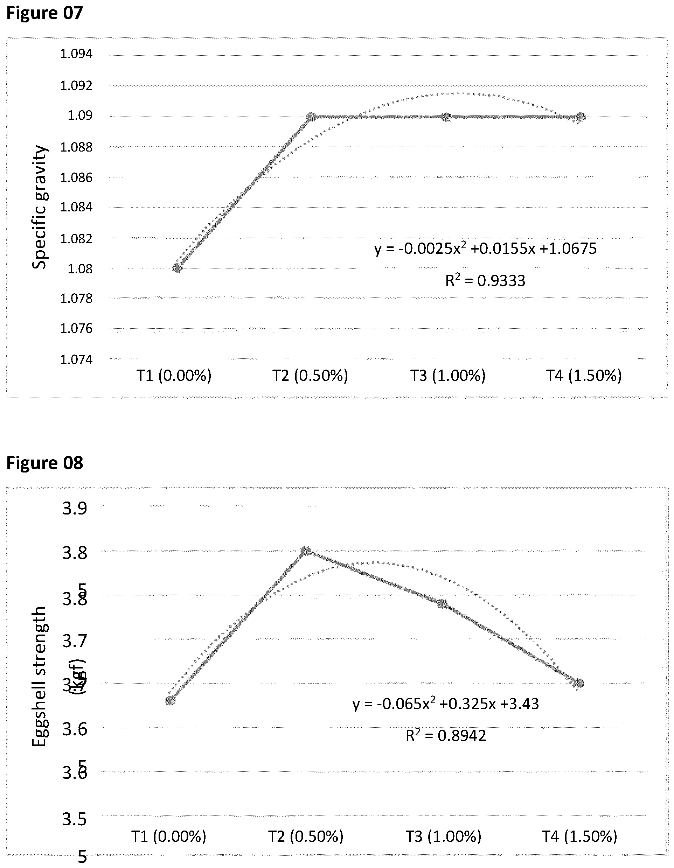

The regression evaluation for the egg quality characteristic followed the same trend as the conventional statistical evaluation, with significant effect only for the characteristics of specific gravity, eggshell resistance, and eggshell percentage, which presented quadratic behavior with better results for inclusion levels between 0.50 and 1.00%, as can be seen in figures 07, 08, and 09.

Before the beginning of the experiment described here and after it ended, blood was collected from 48 birds for assessment of total cholesterol, LDL (low density lipoprotein), triglycerides, HDL (high density lipoprotein), and Tbars (substances reactive to thiobarbituric acid - nmol MDA/mL) in blood serum. Such data is found in Table 25.

Table 25: Total cholesterol, LDL, triglycerides, HDL, and Tbars values in the blood serum of laying hens supplemented with different levels of lignin.

| Treatment | Total cholesterol (mg/dL) | Direct LDL (mg/dl) | Triglycerides (mg/dl) | HDL cholesterol (mg/dl) | Tbars (nmol MDA/mL) |
|---|---|---|---|---|---|
| Initial | 82.43 A | 12.35 C | 1169.67 | 48.71 A | 1.64 A |
| T1 | 81.79 A | 12.71 C | 1275.92 | 44.50 A | 1.63 A |
| T2 | 75.42 C | 16.46 B | 1223.08 | 36.92 B | 1.43 B |
| T3 | 72.50 C | 18.05 A | 1293.83 | 38.00 B | 1.44 B |
| T4 | 77.80 B | 14.38 C | 1233.92 | 34.08 C | 1.50 AB |
| CV % | 3.22 | 5.60 | 1.67 | 4.70 | 4.05 |
| P-value | 0.001 | 0.001 | 0.780 | 0.024 | 0.031 |

Averages followed by different letters in the column differ from each other using the Tukey's test (p <0.05). CV% = coefficient of variation (%)

Note therefore that one day before the beginning of the experiment the following values were found: 82.43 mg/dL of Total Cholesterol (the maximum limit for total cholesterol is accepted at 120 mg/dL), 12.35 mg/dL of LDL, 1,169.67 mg/dL of Triglycerides, 48.71 mg/dL of HDL cholesterol, and 1.64 nmol MDA/ml of Tbars. These values are within normal limits for laying hens between 40 and 80 weeks.

However, at the end of 84 days, i.e., four production cycles, the inclusion of lignin in the birds' diet provided a difference for the characteristics of total cholesterol, LDL, HDL, and Tbars, with a reduction (p <0.05) for all treatments in which lignin was used. The greatest reduction in the level of total cholesterol and HDL was found for 1.50% inclusion of lignin (-13.70% of cholesterol and -42.93% of HDL), the best result for the LDL level was found for the treatment with inclusion of 1.00% of lignin (46.15%), however, for Tbars, the best results were found for birds treated with 0.50 and 1.00% of lignin (-13.89%).

The evaluations regarding total cholesterol and Tbars were also performed for the birds' eggs before the beginning of the experiment at the end of four productive cycles and for 30 days (shelf life). The data is contained in Table 26 below.

Table 26: Total cholesterol and Tbars values in eggs from laying hens supplemented with different levels of lignin.

| Treatment | Total Cholesterol (mg/dL) | Tbars (mg MDA/kg sample) |
|---|---|---|
| Initial | 1450.87 A | 0.123 D |
| T1 | 1470.41 A | 0.575 A |
| T2 | 921.41 D | 0.368 B |
| T3 | 1086.91C | 0.287 C |
| T4 | 1388.67 B | 0.314 BC |
| CV% | 9.89 | 7.84 |
| P-value | 0.01 | 0.02 |

Averages followed by different letters in the column differ from each other using the Tukey's test (p <0.05). CV% = coefficient of variation (%)

The average value of Tbars in eggs at the beginning of the experiment was 0.123 mg MDA/kg, which are adequate values for fresh eggs (acceptable up to 0.540 mg MDA/kg). The data in the table above is for total cholesterol and Tbars at the beginning and at the end of the experiment. Again, the inclusion of 0.50% and 1.00% of lignin is verified to provide better results for total cholesterol levels in egg yolks ($p < 0.05$). The rate of lipid oxidation measured by Tbars decreased in the eggs of birds supplemented with different percentages of lignin inclusion.

Tables 27 to 29 below show the shelf life data for the quality of eggs from the birds in the present study and stored up to 30 days in a refrigerator.

Table 27: Total cholesterol and Tbars values in eggs from laying hens supplemented with different levels of lignin during different storage periods.

| Treatment | Total Cholesterol (mg/dL) | | | | CV % | P-value | Tbars (mg MDA/kg of sample) x days of storage | | | | CV % | P-value |
|---|---|---|---|---|---|---|---|---|---|---|---|---|
| | 0 | 10 | 20 | 30 | | | 0 | 10 | 20 | 30 | | |
| T1 | 1470.41 Ab | 1445.1 7b | 1996.15 Aa | 2089.65 Aa | 8.09 | 0.01 | 0.575 Aa | 0.342 Bc | 0.275 Cb | 0.209 Bc | 6.78 | 0.01 |
| T2 | 921.41 Dc | 1343.56 b | 1744.16 Ba | 1746.99 Ba | 7.99 | 0.01 | 0.368 B | 0.356 A | 0.301 A | 0.226 A | 4.08 | 0.12 |
| T3 | 1086.9 1Cb | 1205.3 3a | 1315.33 Cab | 1500.18 Ca | 8.90 | 0.01 | 0.287 C | 0.338 B | 0.287 BC | 0.194 C | 4.19 | 0.23 |
| T4 | 1388.67 Bb | 1442.60b | 1392.81 Cb | 2026.67 Aa | 7.66 | 0.03 | 0.314 BCa | 0.341 Ba | 0.281 Cb | 0.195 Cc | 5.33 | 0.03 |
| CV% | 9.81 | 8.64 | 8.71 | 9.56 | | | 4.62 | 4.56 | 3.43 | 3.24 | | |
| P-valu e | 0.05 | 0.23 | 0.01 | 0.04 | | | 0.02 | 0.01 | 0.01 | 0.02 | | |

Averages followed by A, B in the column; a, b, c, on the line, differ from each other by the Tukey's test (p <0.05). CV% = coefficient of variation (%)

Table 28: Egg quality parameters of laying hens fed with different levels of inclusion of lignin to the diet

| Treatment | Egg weight (g) | | | | Specific gravity | | | | Eggshell Strength (kgf) | | | |
|---|---|---|---|---|---|---|---|---|---|---|---|---|
| | Days of storage | | | | | | | | | | | |
| | 0 | 10 | 20 | 30 | 0 | 10 | 20 | 30 | 0 | 10 | 20 | 30 |
| T1 | 67.5 9a | 67.5 4a | 67.24 ab | 67.0 0b | 1.08B a | 1.07a | 1.06b | 1.06b | 3.68 Ba | 3.56 Bb | 3.50 Bb | 3.21 Bc |
| T2 | 68.0 0a | 67.8 9a | 67.35 b | 67.0 0b | 1.09A a | 1.07a | 1.06b | 1.05b | 3.85 Aa | 3.66 Aa | 3.61 Aba | 3.47 Ab |
| T3 | 67.9 5a | 67.8 1a | 67.11 b | 65.6 4c | 1.09A a | 1.07a | 1.06b | 1.05b | 3.79 Aa | 3.67 Ab | 3.61 ABb | 3.56 Ab |
| T4 | 68.5 4a | 68.1 6a | 67.82 ab | 67.4 9b | 1.09A a | 1.07a | 1.07a | 1.05b | 3.70 Ba | 3.59 ABb | 3.55 Bb | 3.23 Bc |

Averages followed by A, B in the column; a, b, c, on the line, differ from each other by the Tukey's test (p <0.05).

Table 29: Percent composition parameters for eggs from laying hens fed with different levels of lignin inclusion in the diet.

| Treatment | Yolk (%) | | | | Eggshell (%) | | | | Albumen (%) | | | |
|---|---|---|---|---|---|---|---|---|---|---|---|---|
| | Days of storage | | | | | | | | | | | |
| | 0 | 10 | 20 | 30 | 0 | 10 | 20 | 30 | 0 | 10 | 20 | 30 |
| T1 | 26.60b | 26.62ab | 27.54ab | 28.06a | 8.83 B | 8.76Ab | 8.88 A | 8.99 A | 64.58a | 64.62a | 63.59b | 62.95c |
| T2 | 26.21b | 27.97a | 27.31a | 27.58a | 9.31 A | 8.95 A | 8.72 A | 8.94 B | 64.48a | 63.08b | 63.98b | 63.48b |
| T3 | 26.44b | 27.84ab | 28.41a | 27.61ab | 9.23 A | 8.03 B | 8.65 B | 8.90 B | 64.33a | 64.13a | 62.95b | 63.49b |
| T4 | 26.51b | 27.44ab | 26.73ab | 28.68a | 8.90 B | 8.76AB | 8.78 A | 9.14 | 64.60a | 63.80ab | 64.49ab | 62.19b |

Averages followed by A, B in the column; a, b, c, on the line, differ from each other by the Tukey's test (p <0.05).
Note that the lowest values of Tbars for fresh eggs (day 0) were found for eggs from chickens fed with 1.00 and 1.50% of lignin, although the level of 0.50% inclusion of this product had also provided a lower Tbars value (p <0.05) to eggs from birds fed the control diet (0.00% inclusion), as per Table 27.
The values of this characteristic had distinct behavior between the different levels of inclusion of lignin in relation to storage time. Only treatments with 0.00 and 1.50% of inclusion of the tested product had a difference (p <0.05) in Tbars values over time. This data demonstrates that treatments 2 and 3 (0.50% and 1.00% of inclusion, respectively) had constant lipid oxidation, i.e., without peak lipid oxidation, whereas treatments 1 and 4 (0.00 and 1,50%) allowed high oxidation of yolk lipids at the beginning of the storage period, with a peak up to 10 days.
Thus, it can be inferred that the egg quality from birds fed with diets with 0.50 and 1.00% of lignin inclusion provide better protection against yolk lipid oxidation.
In addition, it can be seen in Table 28 that the egg weight did not differ (p> 0.05) among treatments on the same evaluation day. Between the evaluation periods, egg weight was noted to decrease (p <0.05) after 20 days of storage. The greatest variation in weight was found for treatment 3 (1.00%), which was -3.46%.
The specific gravity also differed among treatments only on day 0 of the evaluation (this behavior was not repeated later). However, this characteristic is noted to worsen (p <0.05) after 20 days of storage.
The resistance of the eggshell was in turn influenced by treatments and storage time, and the treatments with 0.50 and 1.00% of lignin inclusion had the best results throughout the storage period (see Table 28).
It can be seen from Table 29 that the percentage of yolk and albumen in the eggs was affected by the storage time (p <0.05), with a noticeable increase in the percentage of yolk and a decrease in the percentage of albumen. The level of lignin inclusion was not a decisive factor in said changes (p> 0.05). Such changes demonstrate loss of water from the eggs, however this loss of moisture from the eggs was not enough to significantly change the percentage of eggshell during the storage period (p> 0.05). However, at 30 days of storage, treatments with the inclusion of 0.50 and 1.00% showed a lower eggshell percentage, which supports the statement that eggs from birds which consumed diets with these lignin levels had less water loss.

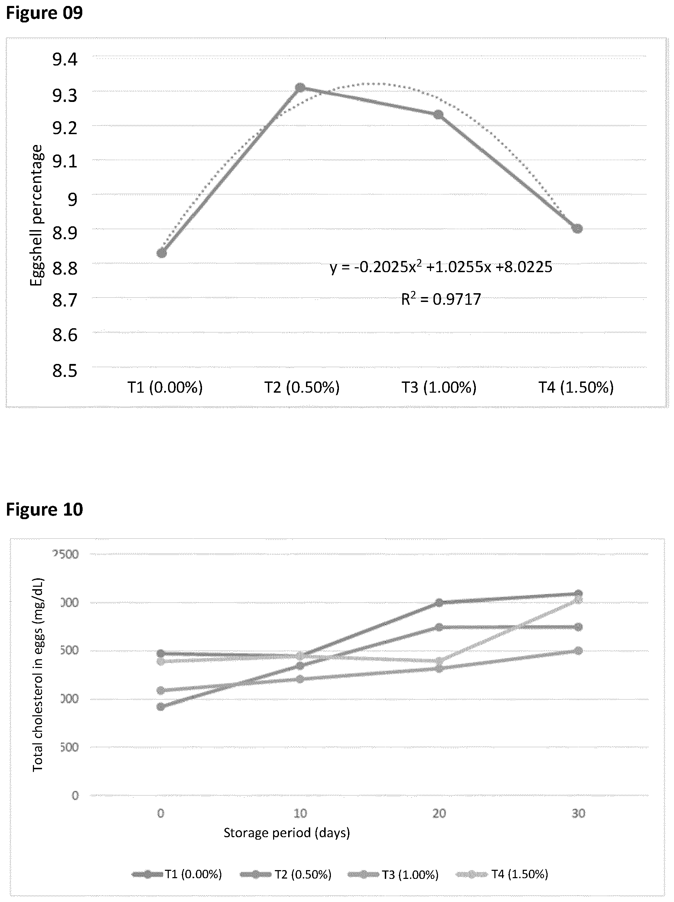

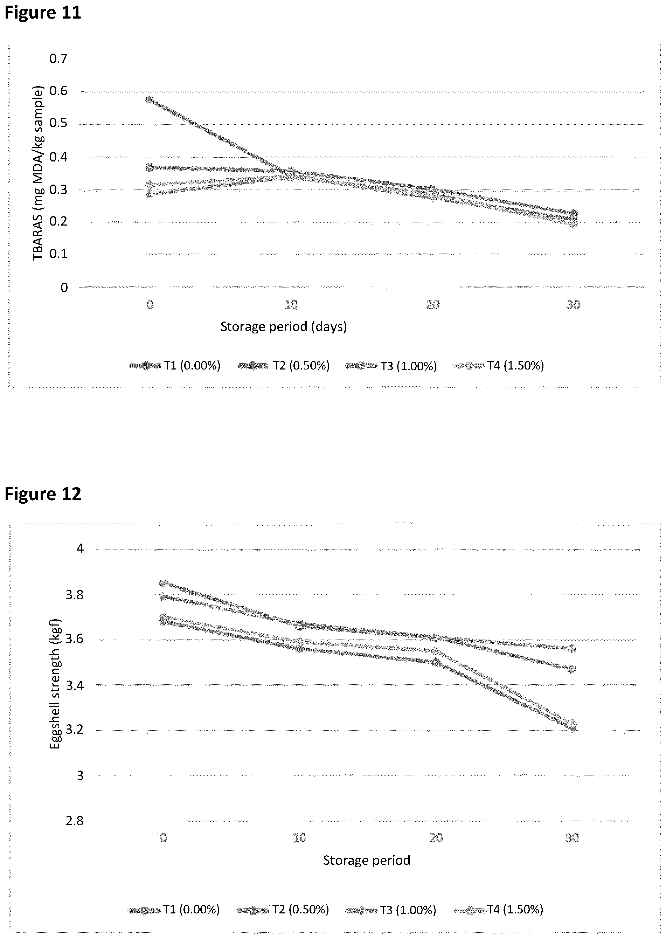

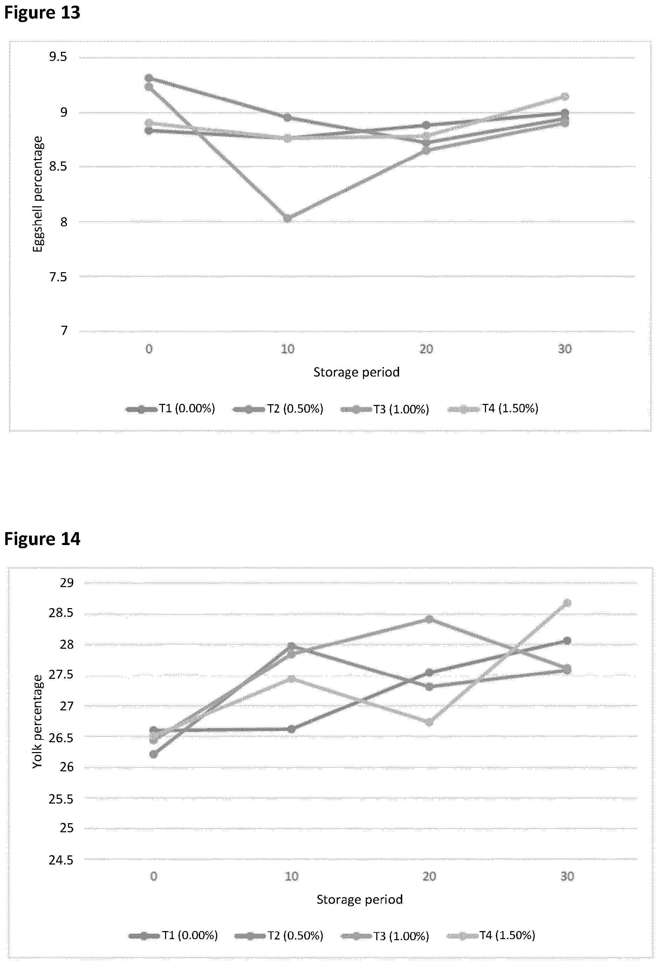

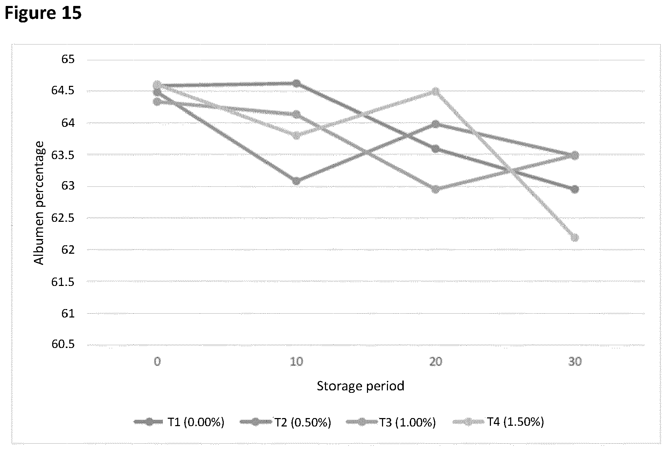

Figures 10 to 15 show graphs demonstrating the behavior of total cholesterol and Tbars levels, as well as the characteristics of eggshell resistance, percentage of eggshell, yolk, and albumen of the eggs during the 30 days of storage.
In the present study, the Intestinal quality of chickens fed with lignin was also evaluated. The data for this analysis is shown in Table 30 below.
The duodenum is known as the portion responsible for most of the digestion and absorption of nutrients. The jejunum is in turn mainly responsible for the absorption of oils, fats, and fat-soluble vitamins. The ileus is the portion most responsible for the absorption of water, minerals, and water-soluble vitamins. Thus, improvements in villi height of these portions are of great importance for improving the birds' performance.
The crypt is responsible for the formation of new intestinal cells. When crypt depth is very high and consequently the villus:crypt ratio is lower, it is understood that some stress agent is promoting the death of active intestinal cells and therefore greater renovation is necessary.
The results found in this experiment were very interesting, as the administration of lignin improved (p <0.05) the intestinal quality for most of the intestinal portions evaluated, especially for Treatment 2 (0.50% of inclusion).

Table 30: Intestinal quality of laying hens supplemented with different levels of lignin.

| Treatment | Length | | | Crypt Depth | | | Villi:Crypt Ratio | | |
|---|---|---|---|---|---|---|---|---|---|
| | Duodenum | Jejunum | Ileus | Duodenum | Jejunum | Ileus | Duodenum | Jejunum | Ileus |
| Initial | 1781.8 B | 1332.0 C | 839.4 C | 254.6 B | 153.0 B | 119.9 B | 6.9 B | 8.7 AB | 7.0 B |
| T1 | 1749.7 B | 1333.7 C | 839.2 C | 253.5 B | 153.1 B | 117.0 B | 6.9 B | 8.7 AB | 7.2 B |
| T2 | 1926.0 A | 1513.2 A | 966.7 A | 268.5 A | 162.2 AB | 125.8 A | 7.2 A | 9.3 A | 7.7 A |
| T3 | 1902.1 A | 1505.0 A | 855.9 C | 266.7 A | 161.2 AB | 116.0 B | 7.2 A | 9.3 A | 7.4 AB |
| T4 | 1681.6 C | 1379.2 B | 884.7 B | 274.2 A | 209.9 A | 118.7 B | 6.1 C | 6.6 B | 7.5 AB |
| CV(%) | 4.10 | 3.62 | 2.55 | 6.13 | 5.67 | 3.31 | 3.87 | 4.23 | 5.01 |
| P-value | 0.01 | 0.01 | 0.01 | 0.01 | 0.01 | 0.01 | 0.03 | 0.01 | 0.03 |

Averages followed by different letters in the column differ by Tukey's test (p <0.05).

It was observed from the study described in the present example embodiment of the invention that the inclusion of lignin to the diets of laying hens is quite interesting. It was possible to conclude from the present study that the 1.00% inclusion level provided the best results for the production of eggs, even though the levels of 0.50 and 1.50% also improved the evaluated characteristics. In this context, the significant improvement in soiled egg percentage stands out, which is a recurring problem for laying hens after the age of 60 weeks.

Egg quality characteristics also improved for eggs from chicken supplemented with lignin, and the most expressive results were found for birds subjected to 0.50 and 1.00% inclusion of this product. The marked improvement in eggshell quality can be highlighted.

The evaluation of shelf life supported the results of improvement in eggshell quality of eggs from birds supplemented with lignin. These data are reinforced by the Tbars results, which were better in eggs from birds supplemented with lignin (0.50 and 1.00% inclusion).

The use of lignin, mainly at 0.50 and 1.00% inclusion, was also efficient in greatly improving the quality of the small intestine, promoting a greater area of nutrient absorption.

## Claims

1. Food composition **characterized in that** it comprises up to 5% by weight of a food additive, wherein the food additive is lignin.

2. Food composition according to claim 1, **characterized in that** it comprises from 1 to 3% by weight of the food additive.

3. Additive composition, **characterized in that** it comprises up to 99% by weight of lignin.

4. Additive composition according to claim 3, **characterized in that** it is a supplement, a concentrate, a premix, or a core.

5. Food composition **characterized in that** it comprises the additive composition defined in claim 3 or 4.

6. Food composition according to any one of claims 1, 2 or 5, **characterized in that** it further comprises components of animal feed.

7. Food composition according to claim 6, **characterized in that** the animal feed components are selected from sugar, flour, bran, starch, sugarcane juice, sugarcane molasses, yeast, powdered milk, oils, sorghum, corn, roasted whole soy, amino acids, limestone, dicalcium phosphate, salt, vitamins, folic acid, choline chloride, antioxidants, and minerals.

8. Food composition according to any one of claims 1 to 7, **characterized in that** the lignin is kraft lignin.

9. Food or additive composition according to claim 8, **characterized in that** kraft lignin is hardwood kraft lignin.

10. Food or additive composition according to claim 9, **characterized in that** the hardwood is eucalyptus wood.

11. Food composition according to any one of claims 1, 2 or 5 to 10, **characterized in that** it is an animal feed.

12. Food composition according to any one of claims 1, 2 or 5 to 11, **characterized in that** it is to improve the animal's zootechnical performance and/or the characteristics of products of animal origin.

13. Food composition according to claim 12, **characterized in that** the improvement of zootechnical performance is selected from the group consisting of increased weight gain, reduced food consumption, reduced feed conversion, reduced fat levels, reduced fat deposition in the abdominal cavity, and histomorphological intestinal changes.

14. Food composition according to claim 13, **characterized in that** reduction in fat levels is a reduction in the levels of LDL, HDL, triglycerides, plasma oxidation, or Tbars.

15. Food composition according to claim 13, **characterized in that** the intestinal histomorphological changes are an increase in crypts depth or an increase in villi height.

16. Food composition according to claim 13 or 15, **characterized in that** intestinal histomorphological changes occur in the jejunum and/or duodenum.

17. Food composition according to claim 12, **characterized in that** the improvement in characteristics in products of animal origin is selected from the group consisting of delaying the *postmortem* oxidative effects in chicken meat, reduced oxidation, and cholesterol in eggs, and increase in resistance and weight of eggshells.

18. Food composition according to any one of claims 1, 2 or 5 to 17, **characterized in that** it is for feeding livestock and companion animals.

19. Use of lignin, **characterized in that** it is as a food additive in a food composition defined in any one of claims 1, 2, 6, 7 or 11 or in an additive composition defined in claim 3 or 4.

20. Use of lignin **characterized in that** it is for preparing a food composition defined in any one of claims 1, 2, 6, 7 or 11 to improve the zootechnical performance of animals and/or the characteristics in products of animal origin.

21. Use according to claim 20, **characterized in that** the improvement of zootechnical performance is selected from the group consisting of increased weight gain, reduced food consumption, reduced feed conversion, reduced fat levels, reduced fat deposition in the abdominal cavity, and histomorphological intestinal changes.

22. Use according to claim 21, **characterized in that** the reduction in fat levels is a reduction in the levels of LDL, HDL, triglycerides, plasma oxidation, or Tbars.

23. Use according to claim 21, **characterized in that** the intestinal histomorphological changes are an increase in crypts depth or an increase in villi height.

24. Use according to claim 21 or 23, **characterized in that** intestinal histomorphological changes occur in the jejunum and/or duodenum.

25. Use according to claim 20, **characterized in that** the improvement in characteristics in products of animal origin is selected from the group consisting of delaying the *postmortem* oxidative effects in chicken meat, reduced oxidation, and cholesterol in eggs, and increase in resistance and weight of eggshells.

26. Use according to any one of claims 19 to 25, **characterized in that** the food composition is for feeding livestock or companion animals.

27. Use of lignin, **characterized in that** it is to prepare an additive composition as defined in claim 3 or 4.

**28.** Use according to any one of claims 19 to 27, **characterized in that** the lignin is kraft lignin.

**29.** Use according to claim 28, **characterized in that** kraft lignin is hardwood kraft lignin.

**30.** Use according to claim 29, **characterized in that** the hardwood is eucalyptus wood.

**31.** Use of the additive composition as defined in claim 3 or 4, **characterized in that** it is for preparing a food composition, as defined in claim 1 or 2, to improve the zootechnical performance of animals and/or the characteristics in products of animal origin.

**32.** Invention of product, process, system, or use, **characterized in that** it comprises one or more elements described in the present patent application.

## Figure 01

p-coumaryl   coniferyl   synaphyl

alcohol     alcohol     alcohol

## Figure 02

**Figure 03**

$y = 1.9667x^3 - 16.725x^2 + 43.418x + 96.26$

$R^2 = 0.99$

**Figure 04**

$y = -0.3625x^2 + 2.6475x + 86.357$

$R^2 = 0.9632$

## Figure 05

## Figure 06

**Figure 07**

$y = -0.0025x^2 + 0.0155x + 1.0675$

$R^2 = 0.9333$

**Figure 08**

$y = -0.065x^2 + 0.325x + 3.43$

$R^2 = 0.8942$

**Figure 09**

**Figure 10**

## Figure 11

## Figure 12

**Figure 13**

**Figure 14**

## Figure 15

## INTERNATIONAL SEARCH REPORT

| International application No. |
| --- |
| PCT/BR2019/050557 |

**A.    CLASSIFICATION OF SUBJECT MATTER**
**IPC: A23K 10/30 (2016.01),  A23K 50/75 (2016.01)**
**CPC: A23K 10/30,  A23K 50/75**
According to International Patent Classification (IPC) or to both national classification and IPC

**B.    FIELDS SEARCHED**

Minimum documentation searched (classification system followed by classification symbols)

**A23K**

Documentation searched other than minimum documentation to the extent that such documents are included in the fields searched

**Base de Patentes INPI-BR; Google; Catálogo de Teses e Dissertações da CAPES**

Electronic data base consulted during the international search (name of data base and, where practicable, search terms used)

**Derwent Innovation; Espacenet; Google Patents**

**C.    DOCUMENTS CONSIDERED TO BE RELEVANT**

| Category* | Citation of document, with indication, where appropriate, of the relevant passages | Relevant to claim No. |
| --- | --- | --- |
| X | BAURHOO,  B.; PHILLIP, L.; RUIZ-FERIA, C. A. Effects of purified lignin and mannan oligosaccharides on intestinal integrity and microbial populations in the ceca and litter of broiler chickens.<br><br>Poultry Science, Urbana, v. 86, p. 1070- 1078, 2007. Disponível em: <https://www.sciencedirect.com/seience/article/pii/S0032579119398 979>, acessado em 27 February. 2020. The whole document.<br><br>--------------------------------------- | 1-7,   11-13,   14 (parte) 15, 16, 17 (parte),    18-21, 23, 22 (parte), 24, 25 (parte), 26, 27, 31 e 32 |

| [X]   Further documents are listed in the continuation of Box C. | [X]   See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 2 March 2020 | 24 March 2020 |

| Name and mailing address of the ISA/BR | Authorized officer |
| --- | --- |
| *I N P I*   Rua Mayrink Veiga n° 9, 6° andar<br>cep: 20090-910, Centro - Rio de Janeiro/RJ | **Cibele Cristina Osawa** |
| Facsimile No.   +55 21 3037-3663 | +55 21 3037-3493/3742<br>Telephone No. |

Form PCT/ISA/210 (second sheet) (January 2015)

## INTERNATIONAL SEARCH REPORT

| International application No. |
|---|
| PCT/BR2019/050557 |

**Box No. II    Observations where certain claims were found unsearchable (Continuation of item 2 of first sheet)**

This international search report has not been established in respect of certain claims under Article 17(2)(a) for the following reasons:

1. [X]  Claims Nos.: 14, 17, 22, 25 (in part)
   because they relate to subject matter not required to be searched by this Authority, namely:

   The subject matter claimed in part of claims 14 and 17 is understood to relate to a reduction in LDL, HDL and triglyceride levels and cholesterol levels as a result of consumption of the composition and, therefore, constitutes a method for treatment of the animal body under PCT Rule 39.1(iv). Similarly, the use, understood to be the consumption, of the composition containing 3% lignin for a "reduction in LDL, HDL and triglyceride levels" (part of claim 22) and a reduction in cholesterol in eggs (part of claim 25) constitutes a method for the treatment of the animal body under PCT Rule 39.1(iv).

2. [ ]  Claims Nos.:
   because they relate to parts of the international application that do not comply with the prescribed requirements to such an extent that no meaningful international search can be carried out, specifically:

3. [ ]  Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. [ ]  As all required additional search fees were timely paid by the applicant, this international search report covers all searchable claims.

2. [ ]  As all searchable claims could be searched without effort justifying additional fees, this Authority did not invite payment of additional fees.

3. [ ]  As only some of the required additional search fees were timely paid by the applicant, this international search report covers only those claims for which fees were paid, specifically claims Nos.:

4. [ ]  No required additional search fees were timely paid by the applicant.  Consequently, this international search report is restricted to the invention first mentioned in the claims;  it is covered by claims Nos.:

**Remark on Protest**         [ ]  The additional search fees were accompanied by the applicant's protest and, where applicable, the payment of a protest fee.
                              [ ]  The additional search fees were accompanied by the applicant's protest but the applicable protest fee was not paid within the time limit specified in the invitation.
                              [ ]  No protest accompanied the payment of additional search fees.

Form PCT/ISA/210 (continuation of first sheet (2)) (January 2015)

## INTERNATIONAL SEARCH REPORT

| | International application No. |
|---|---|
| | PCT/BR2019/050557 |

C (Continuation).  DOCUMENTS CONSIDERED TO BE RELEVANT

| Category* | Citation of document, with indication, where appropriate, of the relevant passages | Relevant to claim No. |
|---|---|---|
| X | RICKE, S. C. et al. Influence of dietary fibres on performance and fermentation characteristics of gut contents from growing chicks. Poultry Science, Urbana, v. 61, p. 1335-1343, 1982. Disponível em:<https://www.seiencedirect.com/science/article/pii/S0032 579119352642>. acessado em 27 February. 2020. The whole document. | 1-13, 14 (parte), 17 (parte), 18-21, 22 (parte), 25 (parte), 26-32 |
| X | WO 2018206852 AI (A ALTO UNIV FOUNDATION SR [FI]) 15 Novembre 2018 (2018-11-15) The whole document. | 1-13, 14 (parte), 17 (parte), 18-21, 22 (parte), 25 (parte), 26-32 |
| A | NASCIMENTO, E. A.; MORAIS, S. A. L. Ozonólise das ligninas organossolve e kraft eucalipto, parte ii: cinética nos meios ácido e básico. Química Nova, v. 21, n. 5, p. 578-583, 1998. Disponível em: <http ://www.seielo ,br/pdf/qn/v21 n5/2928. pdf >, acessado em 2 March.2020. The whole document. | 1 a 32, sendo parte das reiv. 14, 17, 22 e 25 |
| A | US 2004047897 AI (BACKERS THOMAS [DE]) 11 March 2004 (2004-03-11) The whole document. | 1 a 32, sendo parte das reiv. 14, 17, 22 e 25 |
| A | JP 2018110551 A (JUJO PAPER CO LTD) 19 July 2018 (2018-07-19) The whole document. | 1 a 32, sendo parte das reiv. 14, 17, 22 e 25 |

Form PCT/ISA/210 (continuation of second sheet) (January 2015)

**INTERNATIONAL SEARCH REPORT**
Information on patent family members

International application No.

PCT/BR2019/050557

| | | | |
|---|---|---|---|
| WO 2018206852 A1 | 2018-11-15 | CN 110831442 A | 2020-02-21 |
| | | CA 3062610 A1 | 2018-11-15 |
| US 2004047897 A1 | 2004-03-11 | US 8153173 B2 | 2012-04-10 |
| | | AT 403380 T | 2008-08-15 |
| | | AU 1405002 A | 2002-05-27 |
| | | BR 0115310 A | 2003-10-07 |
| | | DE 10056345 A1 | 2002-05-29 |
| | | DE 50114199 D1 | 2008-09-18 |
| | | DK 1333728 T3 | 2008-12-01 |
| | | ES 2309100 T3 | 2008-12-16 |
| | | MX PA03004032 A | 2004-09-10 |
| | | PL 363802 A1 | 2004-11-29 |
| | | WO 0239827 A1 | 2002-05-23 |
| JP 2018110551 A | 2018-07-19 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040241194 A
- US 7150889 B
- DE 10106078
- US 20130164413 A
- WO 2017056275 A

**Non-patent literature cited in the description**

- **J.D. VAN DER KLIS ; E. VINYETA-PUNTI.** *The Potential of Phytogenic Feed Additives in Pigs and Poultry*
- **THAO.** *Effects of Supplementation of Eucalyptus (E. Camaldulensis) Leaf Meal on Feed Intake and Rumen Fermentation Efficiency in Swamp Buffaloes*
- Tabelas brasileiras para aves e suínos: composição de alimentos e exigências nutricionais. UFV, 2011